(19) Europäisches Patentamt
European Patent Office
Office européen des brevets

(11) Veröffentlichungsnummer: **0 199 085 B1**

(12) **EUROPÄISCHE PATENTSCHRIFT**

(45) Veröffentlichungstag der Patentschrift: **08.07.92**

(51) Int. Cl.5: **C07G 17/00**, C12P 1/06, A61K 35/66, G01N 33/534, //(C12P1/06,C12R1:365)

(21) Anmeldenummer: **86103719.0**

(22) Anmeldetag: **19.03.86**

# (54) Jomol, seine Derivate, Verfahren zur Herstellung dieser Verbindungen, ihre Verwendung und Erzeugnis.

(30) Priorität: **25.03.85 DE 3510795**

(43) Veröffentlichungstag der Anmeldung:
**29.10.86 Patentblatt 86/44**

(45) Bekanntmachung des Hinweises auf die Patenterteilung:
**08.07.92 Patentblatt 92/28**

(84) Benannte Vertragsstaaten:
**AT BE CH DE FR GB IT LI LU NL SE**

(56) Entgegenhaltungen:
**EP-A- 0 142 641**
**FR-A- 2 447 932**
**US-A- 4 245 047**

(73) Patentinhaber: **Ehrenfeld, Udo, Dr.**
**Furtmayrstrasse 20**
**W-8400 Regensburg(DE)**

(72) Erfinder: **Ehrenfeld, Udo, Dr.**
**Furtmayrstrasse 20**
**W-8400 Regensburg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 199 085 B1

## Beschreibung

Die Erfindung betrifft Jomol, Jomolderivate, Verfahren zur Herstellung dieser Verbindungen, ihre Verwendung und Jomol oder Jomolderivate enthaltende Erzeugnisse.

Jomol und seine Derivate können als Arzneimittel und als diagnostische Mittel verwendet werden. Sie sind insbesondere als Mittel zur Steigerung der zelligen Abwehr und für die Diagnose und Therapie von malignen Tumoren sowie zur Behandlung von Schwächen der zelligen und humoralen Immunabwehr geeignet. Jomol kann als Träger für diagnostische und therapeutische Stoffe verwendet werden.

Die Diagnose und die Therapie maligner Tumoren ist trotz intensiver Forschung heute noch schwierig. Es ist fast unmöglich, maligne Tumoren im Frühzustand zu erkennen, und bis heute steht kein Verfahren zur Verfügung, mit dem eine Frühdiagnose durchgeführt werden kann. Die Therapie maligner Tumoren ist - wie allseits bekannt ist - nicht zufriedenstellend.

U. Ehrenfeld (Krebsgeschehen 5, 132 ff (1979)) berichtet über die cancerotoxische Wirkung eines Gemisches aus Acetaldehyd und Ethanol. Das Gemisch enthält 3 bis 10 g Acetaldehyd pro 1000 g Ethanol. Es zeigte sich jedoch, daß die Wirkung dieses Gemisches bei der Behandlung von soliden malignen Tumoren wie auch von Metastasen nicht ausreichend ist.

In den deutschen Offenlegungsschriften DE-OS 3 334 751, DE-OS 3 336 583 und DE-OS 3 402 312 (entsprechend der europäischen Patentanmeldung 84 110 118, der japanischen Patentanmeldung 202 859-84 und der US-Patentanmeldung 689 728 [Anmeldetag 4. Januar 1985] und 557 738 [Anmeldetag 2. Dezember 1983] wird ein Mittel zur Diagnose und Therapie von bösartigen Tumoren sowie zur Therapie von Schwächen der zelligen und humoralen Immunabwehr beschrieben, das einen Immunmodulator in und/oder auf Liposomen oder lipidiert oder einen mit einem radioaktiven Strahler, einem Farbstoff oder einem Cytostatikum markierten Immunmodulator enthält. Ein derartiger Immunmodulator wird vorzugsweise mit einem Mittel zusammen verabreicht, welches einen Aldehyd der Formel RCHO, worin R ein Wasserstoffatom oder eine geradkettige oder verzweigte Kohlenwasserstoffgruppe mit 1 bis 4 Kohlenstoffatomen ist, enthält und gegebenenfalls einen Alkohol der Formel $R^1CH_2O$, worin $R^1$ die für R angegebene Bedeutung besitzt, enthält. Die in den oben genannten deutschen Offenlegungsschriften beschriebenen Mittel sind hoch wirksam und besonders für die Therapie und die Diagnose maligner Tumoren geeignet.

Es besteht jedoch ein Bedarf nach Mitteln, die möglichst keinerlei Nebenwirkungen zeigen und sehr einfach herzustellen sind. Die bekannten Mittel besitzen den Nachteil, daß ihre intravenöse Verabreichung in manchen Modifikationen mit gewissen Schwierigkeiten verbunden ist.

Der vorliegenden Erfindung liegt die Aufgabe zugrunde, ein Mittel und ein Erzeugnis für die Diagnose und die Therapie von Tumoren zur Verfügung zu stellen, mit denen man auch die Immunabwehrschwäche bei Menschen und Tieren erfolgreich behandeln kann. Das Mittel soll hoch wirksam sein, daher in geringerer Konzentration als die bekannten Mittel verabreicht werden können, unbegrenzt verfügbar, einfach anzuwenden und gut dosierbar sein. Das erfindungsgemäße Mittel soll bewirken, daß der Organismus des Patienten weniger stark belastet wird. Das Mittel soll weiterhin intravenös verabreichbar sein und als Träger für radioaktive Strahler, Arzneimittel und Farbstoffe dienen.

Überraschenderweise wurde gefunden, daß eine Verbindung, die man aus dem Mikroorganismus Rhodococcus rhodochrous gewinnt, die erfindungsgemäße Aufgabe löst.

Die Erfindung betrifft Jomol, das durch folgende Eigenschaften charakterisiert ist:

(1) Absorptionsmaximum im UV-Spektrum bei 282,5 nm;
(2) apparente Molekulargewichte bei 4500, 3000 und 900;
(3) starke Hygroskopizität;
(4) je nach Restwassergehalt elfenbeinfarbig amorphkristallin-flockig;
(5) pH-Wert von 5,5 (1 mg der Verbindung, gelöst in 1 ml Wasser);
(6) schwach positive, rosa Ninhydrin-Reaktion;
(7) anfärbbar mit Orcin Reagens;
(8) Peptidanteil: ca. 50%, bezogen auf die amorphe Sub stanz
(Proteintest MicroBioRad nach L. Thomas, J. Clin. Chem. Clin. Biochem., Bd. 19, 1981, Seite 203-208, Fa. BioRad, München; Coomassie Brilliant Blue G 250®);
(9) folgende $R_f$-Werte (Lösungsmittelangabe Volumen:Volumen)

| | |
|---|---|
| $R_f$ = 0,20 | (Butanol:Eisessig:Wasser 4:1:1) |
| $R_f$ = 0,45 | (Benzol:Eisessig:Wasser 2:1:2) |
| $R_f$ = 0,685 | (Methanol:Eisessig:Wasser 4:1:1) |
| keine Wanderung | (Chloroform:Methanol 96:4) |
| $R_f$ = 0,54 | (Chloroform:Methanol 1:1); |

(10) Löslichkeit: schlecht löslich in unpolaren Lösungsmitteln, sehr gut loslich in polaren Lösungsmitteln;
(11) nachweisbare Bestandteile:

| Substanzen | Verhältnis (Näherung) |
|---|---|
| Neutralzucker (Glucose, Galactose, Ribose) | (≤) 1 |
| Aminozucker (Glucosamin, Muraminsäure) (nach Hydrolyse) | 3,5 |
| Aminosäuren (Ala, Glu, iso-Gln, Gly, Lys, DAP) | 4 |
| Lipide | <1 |
| Phosphor | <1 |

(12) Struktur:
Peptidoglycanstruktur, deren Glycangerüst aus 10-80 Disacchariden aus N-Acetylglucosaminyl-N-acetylmuramyl besteht,
wobei gilt:

| | | | |
|---|---|---|---|
| Ala | Alanin | Lys | Lysin |
| Glu | Glutaminsäure | DAP | meso-α,ε-Diaminopimelinsäure |
| Gly | Glycin | | |
| iso-Gln | iso-Glutamin | | |

Die Erfindung betrifft weiterhin ein Verfahren zur Herstellung von Jomol durch Züchten eines Stammes von Nocardia-opaca-Zellen und Gewinnen der Zellen aus der Kultur. Das erfindungsgemäße Verfahren ist dadurch gekennzeichnet, daß man
(a) Nocardia-opaca-Zellen (DSM 43 202 bzw. ATCC 21953) auf einem gepufferten Nährmedium züchtet,
(b) die Zellen gewinnt,
(c) die Zellen in einem Glucose und Puffer enthaltenden Medium suspendiert, die Suspension 15 Minuten bis einige Stunden stehen läßt,
(d) die Suspension in einem Puffer mit einem murolytischen Enzym, vorzugsweise Lysozym, und gegebenenfalls mit Desoxyribonuclease behandelt,
(e) die Zellen zerstört,
(f) das erhaltene Material in Bodensatz und Überstand trennt,
(g) den Überstand an Sephadex-Säulen unter Verwendung eines Puffers als Elutierungsmittel, der gegebenenfalls 0,1 bis 0,05% EDTA-$Na_2$ enthalten kann, der Trennung unterwirft,

(h) die aktive Fraktion gewinnt und

(i) die aktive Fraktion mit Acetaldehyd umsetzt,

wobei als Puffer Kaliumphosphatpuffer, Tris-HCl-Puffer, Ammoniumacetatpuffer und andere verwendet werden können.

Weiterhin betrifft die Erfindung en pharmazeutisch annehmbares Jomolderivat, das dadurch gekennzeichnet ist, daß es das oben beschriebene Jomol und daran gekoppelt einen Kronenether, Diethylentriaminpentaessigsäure, Uridin, L-Thyronin, L-Tyrosin, Fluoresceinisothiocyanat oder Zinn(II)-chlorid enthält.

Die Erfindung betrifft außerdem markiertes Jomol oder ein markiertes Jomolderivat, das dadurch gekennzeichnet ist, daß es das vorstehend beschriebene Jomol oder eines seiner pharmazeutisch annehmbaren Derivate, wie oben genannt, markiert mit $^{99m}$Technetium, $^{111}$Indium, $^{123}$Jod, $^{125}$Jod, $^{130}$Jod, $^{131}$Jod, $^{132}$Jod oder $^{224}$Radium, einem Farbstoff oder einem Cytostatikum, enthält. Als Kopplungsmittel können alle Kopplungsmittel verwendet werden, die einen radioaktiven Strahler, einen Farbstoff oder ein Cytostatikum tragen oder binden können.

Die Erfindung betrifft weiterhin Jomol, wie oben beschrieben, ein pharmazeutisch annehmbares Jomolderivat, wie oben beschrieben, markiertes Jomol oder ein markiertes Jomolderivat, wie oben beschrieben, in und/oder auf Liposomen oder lipidiert.

Außerdem betrifft die Erfindung ein diagnostisches Mittel, welches Jomol oder ein Jomolderivat, wie oben beschrieben, markiert mit einem Radioisotop und/oder einem Farbstoff, sowie gegebenenfalls übliche Trägerstoffe und/oder Verdünnungsmittel enthält, und ein Arzneimittel, das Jomol oder ein Jomolderivat, markiert mit einem Farbstoff, einem Cytostatikum oder einem Radioisotop, sowie gegebenenfalls übliche Trägerstoffe und/oder Verdünnungsmittel enthält.

Schließlich betrifft die Erfindung ein Erzeugnis, enthaltend

(1) ein diagnostisches Mittel oder ein Arzneimittel, wie vorstehend beschrieben, und

(2) ein Hilfsmittel, welches einen Aldehyd der Formel I

$$RCHO \quad (I)$$

worin R ein Wasserstoffatom und eine geradkettige oder verzweigte Kohlenwasserstoffgruppe mit 1 bis 4 Kohlenstoffatomen bedeutet, wobei der freie Aldehyd auch direkt oder indirekt von Stoffen metabolisch freigesetzt werden kann, und

gegebenenfalls einen Alkohol der Formel II

$$R^1CH_2OH \quad (II)$$

worin $R^1$ die oben für R angegebene Bedeutung besitzt, sowie gegebenenfalls übliche pharmazeutisch annehmbare Trägerstoffe und/oder Verdünnungsmittel enthält,

zur gleichzeitigen, getrennten oder zeitlich abgestuften Verwendung bei der Diagnose und Therapie von malignen Tumoren und zur Therapie von Immunabwehrschwächen.

Der Anmelder hat überraschenderweise gefunden, daß ein bestimmter Mikroorganismus des Genus Nocardia opaca, nämlich Rhodococcus rhodochrous, eine Verbindung ergibt, die nach Umsetzung mit Acetaldehyd überraschende Eigenschaften als Mittel zur Steigerung der zelligen Immunabwehr und als Träger für diagnostische und therapeutische Stoffe aufweist. Der Stamm Rhodococcus rhodochrous wurde bei der Deutschen Sammlung von Mikroorganismen, Griesbachstraße 8, D-3400 Göttingen, am 16. Mai 1972 unter der Nummer DSM 43 202 (= DSM 363 = ATCC 21 953) hinterlegt. Der Stamm ist frei verfügbar, und seine Lebensfähigkeit wurde am 28. Februar 1985 erneut nachgewiesen.

Bei der Durchführung des erfindungsgemäßen Verfahrens wird ein eine Kohlenstoffquelle, eine Stickstoffquelle und Mineralien enthaltendes Kulturmedium verwendet. Dieses Kulturmedium kann auch Antischaummittel und/oder andere übliche Komponenten enthalten. Beispiele für Kohlenstoffquellen sind Kohlenhydrate, Alkohole, Kohlenwasserstoffe und Kleie. Beispiele für Stickstoffquellen sind Maisquellwasser, Hefeextrakte, Fleischextrakte, Pepton, Fischmehl, Ammoniumsalze, Nitratsalze und Harnstoff. Die Mineralquelle umfaßt anorganische Salze, wie Phosphate, Magnesiumsalze, Zinksalze, Calciumsalze, Mangansalze, Molybdänsalze und Kupfersalze.

Die Zusammensetzung des Kulturmediums kann nach Bedarf geändert werden, und während der Fermentation können diese Kohlenstoff-, Stickstoff- und Mineralquellen zusätzlich zugegeben werden. Zur Herstellung eines Inokulums wird der Mikroorganismus beispielsweise auf DST-Oxoid (Nährboden, Fa. Oxoid, Wesel, FRG) oder Traubenzuckeragar (2%) in Plattentechnik auf drei bis vier Platten fünf bis acht Tage kultiviert. Man erhält hierbei ein Produkt, das dann als Inokulum zur Herstellung des Materials in technischem Maßstab verwendet werden kann.

Die Züchtung des Mikroorganismus in technischem Maßstab kann beispielsweise in Erlenmeyer-Kolben, die 100 ml normale Nährbouillon enthalten, erfolgen. Die Züchtung erfolgt unter aeroben Bedingungen.

Die Züchtungstemperatur beträgt 20 bis 40 ° C, und der pH-Wert des Kulturmediums liegt bei 7,2 bis 7,4. Die bevorzugte Temperatur beträgt 30 bis 37 ° C, und die geeignete Züchtungszeit beträgt normalerweise 2 bis etwa 30 Tage und kann auf geeignete Weise, abhängig von der Auswahl der anderen Kulturbedingungen, geändert werden.

Die Bakterien werden dann geerntet, vorzugsweise durch Abzentrifugieren. Man kann die Bakterien jedoch auch über Glasfilter abfiltrieren. Das Zentrifugieren kann beispielsweise mit 4000 Umdrehungen pro Minute während einer Zeit von 5 bis 10 Minuten in einer Roto-Silenta-K-Hettich-Kühlzentrifuge mit einem Rotor von 40 cm Durchmesser erfolgen. Die erhaltenen Bakterien werden üblicherweise gewaschen, beispielsweise mit Wasser oder einem Puffer, wie beispielsweise 0,01 M Tris-HCl-Puffer mit einem pH-Wert von beispielsweise 7,4. Der Tris-HCl-Puffer enthält vorzugsweise EDTA-$Na_2$, zum Beispiel 0,1 bis 0,04, vorzugsweise 0,06%. Der Waschvorgang kann einmal oder mehrere Male wiederholt werden. Beispiele für andere verwendbare Puffer sind Kaliumphosphatpuffer oder Ammoniumacetatpuffer. Es ist ein besonderer Vorteil des erfindungsgemäßen Verfahrens, daß durchgehend, d.h. bei allen Stufen, der gleiche Puffer verwendet werden kann.

Die Bakterien werden sodann, vorzugsweise nachdem sie wie oben gewaschen wurden, in Tris-HCl-Puffer (pH 7,4), beispielsweise 0,01 M, der vorzugsweise 0,1 bis 0,05% EDTA-$Na_2$ und 8 bis 12%, vorzugsweise 10%, Glucose enthält, oder einem anderen der oben angegebenen Puffer suspendiert.

Zu der erhaltenen Suspension, die gegebenenfalls bebrütet wurde, wird dann ein murolytisches Enzym, vorzugsweise Lysozym (Lysozyme, Sigma L-6876®, Sigma, München), und vorzugsweise zur Entfernung der Nucleinsäuren und zur Herabsetzung der Viskosität Desoxyribonuclease (Deoxyribonuclease, Sigma D-0876®) zugesetzt. Man verwendet beispielsweise für 5 g Bakterien 100 ml Tris-HCl-Puffer und gibt dann zu dieser Suspension 10 mg Lysozym und 3 mg Desoxyribonuclease.

Die Behandlung mit Glucose und den Fermenten wird während einer Zeit von 30 Minuten bis mehreren Stunden, vorzugsweise während einer Zeit von zwei bis drei Stunden, bei einer Temperatur von 20 bis 40 ° C, vorzugsweise 30 bis 37 ° C, durchgeführt.

Das Reaktionsgemisch wird dann während einer Zeit von 10 Minuten bis mehreren Stunden stehen gelassen.

Alternativ kann das Reaktionsgemisch auch während einer Zeit von 10 Minuten bis mehreren Stunden, vorzugsweise 20 Stunden, bei einer Temperatur von 30 bis 37 ° C bebrütet werden. Wird eine Bebrütung durchgeführt, erfolgt anschließend eine Trennung in Bodensatz und Überstand. Beispielsweise erfolgt eine derartige Trennung durch Abzentrifugieren. Der Bodensatz wird dann erneut in Tris-HCl-Puffer (pH 7,0, vorzugsweise 0,01 molar), der vorzugsweise 0,06% EDTA-$Na_2$ enthält, oder einem anderen der oben genannten Puffer resuspendiert. Gegebenenfalls kann er mit diesem Puffer auch einmal oder mehrere Male gewaschen werden. Der Waschvorgang kann unterbleiben, jedoch wird durch ihn mehr Wandmaterial der Bakterien aus dem abschließend verwendeten Überstand entfernt.

Anschließend werden die Zellen zerstört. Dies kann mit allen geeigneten Methoden geschehen, beispielsweise Ultraschall.

Vorzugsweise wird Ultraschall während beispielsweise einer Minute, zum Beispiel mit dem "Sonicator cell disruptor" von Kontron Ultrasonics, Modell W 185 F®, 15 mm Arbeitsende, Stufe 3, Skala bei 50, verwendet. Die mechanische Zerstörung der Zellen muß nicht durchgeführt werden, sie wird jedoch vorzugsweise durchgeführt, weil dadurch die Ausbeute an dem gewünschten Produkt erhöht wird.

Das erhaltene Material wird danach in Überstand und Bodensatz, vorzugsweise durch Zentrifugieren, getrennt. Das Zentrifugieren kann in einer normalen Zentrifuge durchgeführt werden, bevorzugt wird es jedoch in einer Kühlzentrifuge bei einer Temperatur von 4 bis 6 ° C bei 4000 Umdrehungen pro Minute 10 bis 15 Minuten lang durchgeführt. Der Bodensatz kann gegebenenfalls einmal oder mehrere Male mit Wasser oder einem der oben erwähnten Puffer gewaschen werden, wobei die erhaltenen Waschlösungen mit dem Überstand vereinigt werden. Der Überstand wird gegebenenfalls nach Einengen im Hochvakuum bei tiefen Temperaturen an Sephadex-G-75-Säulen chromatographiert. Wird auf ein Waschen des erhaltenen Bodensatzes verzichtet, so wird der Überstand direkt der Chromatographie, ohne daß er eingeengt wird, unterworfen.

Man erhält beispielsweise aus 5 g Bakterien einen Überstand, den man vorzugsweise in fünf gleiche Teile teilt und auf fünf vorbereitete Sephadex-G-75-Säulen zu je einem Teil gibt. Die Säulen sind 80 cm lang, der Innendurchmesser beträgt 2,5 cm, und die Sephadex-Füllungshöhe beträgt 60 cm. Wird nur eine Säule verwendet, so wird der Teil, der nicht chromatographiert wird, bei -25 ° C eingefroren und bei dieser Temperatur aufbewahrt.

Wenn man bei der Chromatographie auf einer der Säulen zur Analytik der Substanz "Albumin aus

Humanserum Markierung" (Bestellnr. 11885, Serva, Heidelberg, FRG) benutzt, tritt der erste Peak der Rohsubstanz mit dem Albumin (MG 69 000) und die Fraktion 2b* mit dem ebenfalls zur Säuleneichung benutzten Vitamin $B_{12}$ (MG 1 355,4) aus (vgl. Figur 3b).

Als Elutionsmittel wird 0,01 M Tris-HCl-Puffer (pH 7,4), der 0,06% EDTA-$Na_2$ enthält, verwendet. Die Laufgeschwindigkeit beträgt 100 $\mu$l pro Minute, wobei man auch niedrigere oder höhere Geschwindigkeiten anwenden kann. Die aktive Fraktion, die als Fraktion 2b* bezeichnet wird, wird in einer Zeit von 12 bis 14 Stunden nach Beginn der Gelchromatographie gesammelt. Das Elutionsvolumen beträgt ca. 900 bis 980 ml, und die Fraktionierung erfolgt unter UV-Detektion bei 214 nm (vgl. Fig.3a). Das Vorgehen zur Gewinnung der Fraktion 2b* kann vom Abernten der Zellen nach Kultivieren bis zur Säulenfraktionierung auch in Phosphatpuffer, Ammoniumacetatpuffer und anderen erfolgen. Die Verwendung von Ammonimacetatpuffer ist insbesondere für die Präparate zur Jodierung bevorzugt.

Die erhaltenen aktiven Fraktionen werden lyophilisiert und anschließend eine Stunde bei 80 ° C zur Zerstörung der Proteasen inkubiert. Wenn nicht lyophilisiert wird, werden die verdünnten Lösungen bei -20 ° C, vorzugsweise -40 ° C, besonders bevorzugt -80 ° C, eingefroren und als Lösung aufbewahrt. Vor dem Lyophilisieren bzw. Einfrieren werden die erhaltenen Lösungen der aktiven Fraktionen mit einer 1 M Acetaldehyd(reinst)-wäßrigen Lösung im unten genannten Molverhältnis gemischt und 10 Minuten bis 2 Stunden, vorzugsweise zum Beispiel 30 Minuten, bei Raumtemperatur stehen gelassen.

Überraschenderweise zeigte sich, daß man bei der Umsetzung der aktiven Fraktion 2b* mit Acetaldehyd eine "Fraktion 2b" erhält, die als Jomol bezeichnet wird und überraschende pharmakologische Eigenschaften aufweist und außerdem als Trägersubstanz verwendet werden kann.

Die Umsetzung der Fraktion 2b* mit Acetaldehyd erfolgt im molaren Verhältnis Fraktion 2b* : Acetaldehyd = 1 : 1,8 bis 2, vorzugsweise im Verhältnis 1 : 2, wobei man für die Fraktion 2b* ein mittleres Molekulargewicht von ca. 4000 annimmt. Wie oben erläutert, werden bei dem erfindungsgemäßen Verfahren Nocardia-Bakterien verwendet. Nocardien sind grampositive Bakterien. Ihr mehrschichtiges Zellwandgrundgerüst weist Einlagerungen von Proteinen und Polysacchariden auf. Ihm aufgelagert sind Deckschichten, die schwer ablösbar sind.

Die Bakterienwand enthält als äußere Schicht Lipoproteine (unter anderem auch Wachse), als mittlere Schicht Lipopolysaccharide und als innere Schicht Polysaccharidketten (Mureingerüst). Die Antigenität der Schichtbestandteile nimmt von außen nach innen ab.

Das Substrat für die Herstellung von Jomol ist die innere Schicht mit den langen Peptidoglykanketten. Diese sind im Glykangerüst aus Disaccharidbausteinen gebildet. Der Disaccharidbaustein besteht aus N-Acetylglucosaminyl-N-acetylmuramyl:

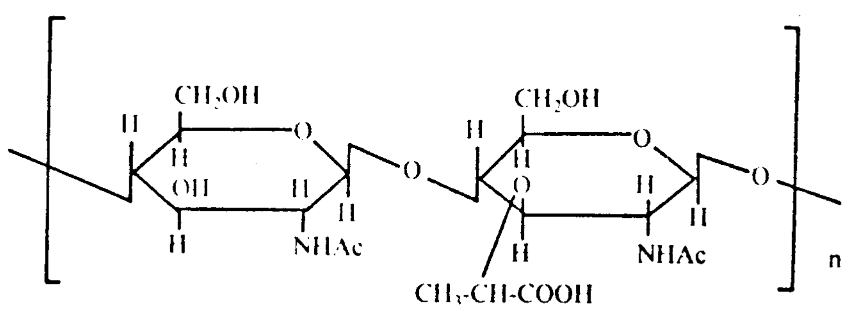

An der Säuregruppe der N-Acetylmuraminsäure (N-Acetylglucosaminlactatether) sind Peptideinheiten über L-Alanin gebunden. Diese Peptideinheiten können Sequenzen von L-Alanin - D-Isoglutamin - meso-$\alpha$,$\epsilon$-Diaminopimelinsäure (DAP) enthalten, wobei die DAP amidiert sein kann. An ihrer Stelle kommt selten Uridindiphosphat oder eine andere Aminosäure, häufiger L-Lysin vor, das seinerseits substituiert sein kann. Anstelle der Acetylgruppe kann an der Muraminsäure eine Glykolylgruppe vorhanden sein. Außer diesen genannten Einheiten können auch 0 bis 10% Neutralzucker vorhanden sein.

Bei dem obigen Herstellungsverfahren wird der Anteil der äußeren Wandschicht der Nocardiabakterien verworfen. Peptidoglykane mit Lipiden und Wachsen sind also weder im Gesamtpräparat noch im Präparat Fraktion 2b enthalten. Die hergestellten Präparate sind Spaltprodukte von Peptidoglykanen der inneren Wandschicht. Das zur Spaltung eingesetzte Lysozym hydrolysiert die Bindung zwischen N-Acetylglucosamin und N-Acetylmuraminsäure des Glykangerüstes. Die Desoxyribonuclease setzt die Viskosität des Produkts herab. Die interpeptidischen Bindungen der Peptidsubstituenten der Peptidoglykane werden

teilweise gespalten, so daß sie wasserlöslich werden und nur noch gering fettlöslich sind. Gleichzeitig verlieren die Enden der Peptidoglykanketten ihre Peptidanteile. Durch den Herstellungsvorgang werden die Peptidoglykane weitestgehend von natürlichen Lipiden, mit denen sie zusammen in den Bakterienwänden vorkommen, befreit. Die N-Acetylglucosamingruppen der Peptidoglykane können teilweise desacetyliert sein.

Die Ausbeute aus ca. 5 g Bakterien beträgt ca. 2 mg Jomol in reiner Form.

Das Produkt ist sehr hygroskopisch. Wägung unter Feuchtigkeitszutritt (Luft) erlaubt keine exakten Substanzmengenangaben; deshalb wird im vorher gewogenen verschlossenen Gefäß nach der Lyophilisierung gewogen. Gegebenenfalls kann mit üblichen Methoden eine Wassergehaltsbestimmung des Lyophilisats durchgeführt werden.

Nach dem Trocknen ist die Substanz gelbweiß und flockig, mit etwas Wasser erscheint sie im Augenblick "kristallin"; mit etwas mehr Wasser ist sie farblos, glasartig, gelatinös und zieht Fäden wie Haushaltsalleskleber. Bei steriler Lagerung ohne Lichtzutritt unter Stickstoff ist die Substanz bei einer Temperatur unter -25°C stabil. Die Substanz ist unter Lichtzutritt bei Raumtemperatur einige Stunden stabil.

Jomol ist flüchtig, außer bei 0°C in Lösungen. Im gefriergetrockneten Zustand wurde dieses Verhalten der Substanz nicht beobachtet. Wird Jomol oder ein es enthaltendes Produkt zum Beispiel über fünf Tage bei Raumtemperatur in wäßriger Lösung in einem Kunststoffgefäß aufbewahrt, so ist ein Verlust von ca. 50% der Substanz festzustellen. Der Effekt tritt in gefrorenem Medium nicht auf.

Jomol besitzt unerwartete Eigenschaften. Jomol selbst findet als Immunmodulator, Arzneimittel oder Diagnostikum Verwendung. Jomol und seine Kopplungsprodukte mit bestimmten Kopplungsmitteln eignen sich besonders gut als Trägersubstanzen für radioaktive Verbindungen, Farbstoffe und Arzneimittel, vorzugsweise Cytostatika.

Jomol, seine Kopplungsprodukte und die markierten oder beladenen Jomolderivate reichern sich überraschenderweise an Krebszellen an und werden von gesunden Zellen kaum oder nur in geringem Umfang gebunden. Man nimmt an, daß es an Spaltprodukte der Krebszellenwandmatrix gebunden wird, die durch Protease gebildet werden. Wird beispielsweise Jomol mit einem radioaktiven Marker markiert und verabreicht, so reichert sich das markierte Jomol an den Tumorzellen an, und diese können dadurch mittels Gammakamera-Imaging leicht nachgewiesen werden.

Beispiele für Kopplungsprodukte von Jomol sind Produkte, die Jomol, daran gekoppelt einen Kronenether, Diethylentriaminpentaessigsäure, Uridin, L-Thyronin, L-Tyrosin, Fluoresceinisothiocyanat oder Zinn(II)-chlorid enthalten. Derartige Kopplungsprodukte werden allgemein hergestellt, indem man Jomol in einem geeigneten polaren Lösungsmittel oder in einer wäßrigen Lösung eines polaren Lösungsmittels löst. Beispielsweise kann man 1 bis 10 mg Jomol in 5 bis 200 µl eines geeigneten wäßrigen Lösungsmittels lösen. Man kann auch sehr konzentrierte Lösungen von Jomol herstellen. Die Lösung erfolgt bei möglichst tiefer Temperatur, wobei jedoch die Temperatur so gewählt werden muß, daß das Lösungsmittel noch nicht kristallisiert. Die mit Jomol umzusetzende Substanz wird in einem Verhältnis von 1 Mol Jomol zu 0,8 bis 1,2 Mol der anzukoppelnden Verbindung umgesetzt, wobei man für das Jomol ein mittleres Molekulargewicht von ca. 4000 annimmt. Die Verbindung, die an Jomol angekoppelt wird, wird in einem wasserfreien Medium gelöst oder homogen suspendiert. Im Falle von Zinn(II)-chlorid wird eine salzsaure wäßrige Lösung verwendet.

Das Reaktionsgemisch wird während einer Zeit von einigen Minuten bis 60 Minuten bei einer Temperatur im Bereich von 15 bis 40°C stehengelassen. Das Produkt kann direkt verwendet werden. Es kann auch durch Chromatographie gereinigt und isoliert werden.

Chromatographie und Reinigung erfolgen auf ähnliche Weise, wie für Jomol selbst beschrieben. Es werden Sephadex-Säulen verwendet, und als Eluierungsmittel werden die oben erwähnten Puffer eingesetzt.

Als aktive Fraktionen werden Fraktionen gesammelt, die in ml 5 bis 10 aus einer 25 cm langen, Durchmesser: 0,6 cm, Säule, Sephadex G75 Füllhöhe: 15 cm, gleichmäßige ElutionsmittelÜberschichtung 8 cm über Sephadexhöhe, voräquilibriert mit den gewünschten Fraktionen, austreten oder die bei UV-Detektion bei 214 nm den ersten beiden Peaks von Jomol entsprechen.

Werden die Kopplungsprodukte selbst direkt als Diagnostikum oder als Arzneimittel verwendet, ist es bevorzugt, möglichst reine Lösungen umzusetzen und die erhaltenen Lösungen direkt als Arzneimittel, gegebenenfalls nach Sterilisation, zu verwenden.

Es wurden folgende Kopplungsprodukte hergestellt:

1. Kopplungsprodukt aus Jomol und Diethylentriaminpentaessigsäureanhydrid

Dieses Produkt wird als "Jomol-DTPA" oder als "Jomo-In" bezeichnet. Es eignet sich zur Beladung mit $^{111}$Indium oder mit $^{99m}$Technetium, welches mit Zinn(II)-chlorid reduziert wurde.

Die mit einem radioaktiven Marker markierten Produkte sind insbesondere für die Diagnostik

geeignet.

2. Kopplungsprodukt aus Jomol und Zinn(II)-chlorid

Dieses Produkt wird als "Jomol-Sn" oder "Jomo-tech" bezeichnet. Jomol-Sn ist insbesondere für die Beladung mit $^{99m}$Technetium geeignet.

Das markierte Produkt ist für die Diagnose von Tumoren besonders gut geeignet.

Das Produkt wird durch Umsetzung einer Jomollösung mit einer salzsauren, wäßrigen Zinn(II)-lösung hergestellt.

3. Kopplungsprodukt aus Jomol und Uridin

Dieses Produkt wird als "Jomo-J/U" bezeichnet und ist zur Markierung mit radioaktivem Jod besonders geeignet. Das markierte Produkt kann zur Diagnose oder Therapie verwendet werden.

Die Herstellung erfolgt durch Umsetzung von Jomol und Uridin-2',3'-dialdehyd in einem wasserfreien Lösungsmittel.

4. Kopplungsprodukt aus Jomol und L-Thyronin

Das Produkt wird als "Jomo-J/Tn" bezeichnet und durch Umsetzung von Jomol mit α-Amino-β-[p-hydroxyphenyl-(p-hydroxyphenylether)]-propionaldehyd hergestellt.

5. Kopplungsprodukt aus Jomol und L-Tyrosin

Das Produkt wird als "Jomo-J/TS" bezeichnet und wird durch Umsetzung von Jomol mit α-Amino-β-[p-hydroxyphenyl]-propionaldehyd hergestellt.

6. Kopplungsprodukt aus Jomol und Fluoresceinisothiocyanat

Das Produkt wird als "Jomo-Color" bezeichnet. Es eignet sich insbesondere zur Diagnostik und/oder Therapie.

Die Produkte 4, 5 und 6 sind besonders gut für die Markierung mit radioaktivem Jod geeignet. Die produkte werden für die Diagnostik und Therapie verwendet.

7. Kopplungsprodukt aus Jomol und einer Vorstufe von Kryptofix 222

Dieses Produkt wird als "Jomo-CE" und auch als "JomoRa" bezeichnet. Das Produkt wird hergestellt, indem Jomol mit einer Vorstufe von Kryptofix 222®, die von der Firma Merck, Darmstadt, erhä1tlich ist und als CE bezeichnet wird, umgesetzt wird. Das CE-Molekül besitzt die folgende Struktur:

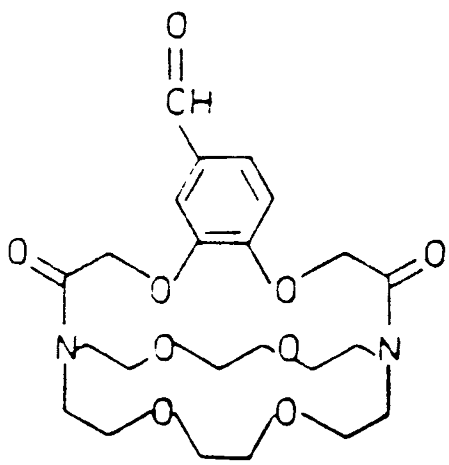

CE enthält die komplette Amidbindung im Kronenether (im Gegensatz zum Kryptofix 222).

Die Verbindung ist besonders für die radioaktive Markierung mit Radium geeignet. Es ist eine längere Inkubationszeit mit $^{224}$Ra zu dessen Aufnahme nötig als beim Kryptofix 222. Die Stabilitätskonstante hinsichtlich der Radiumbindung ist für CE und Kryptofix 222 gleich: lg K = 6,64 (dieser Logarithmus ist für Radium höher als für alle in Frage kommenden anderen Metallionen). Das angebundene Radium kann hinsichtlich seiner Bindung nur durch ein saures Milieu abgelöst werden (im Magen instabil, im übrigen Körper stabil). Ansäuerungen müssen nach der Bindung des Radium an CE unterbleiben.

Die markierte Verbindung ist für die Therapie geeignet.

Jomol und seine oben beschriebenen Kopplungsderivate werden als Trägersubstanzen für Farbstoffe, Arzneimittel, vorzugsweise Cytostatika, und radioaktive Marker verwendet. Da sich diese Substanzen, wie oben erwähnt, an Krebszellen anreichern, ist es somit möglich, die Krebszellen gezielt nachzuweisen oder gezielt zu behandeln. Es ist dadurch möglich, die Wirkstoffe, wie beispielsweise die radioaktiven Marker, die

einerseits zum Nachweis der Krebszellen und andererseits auch zur Zerstörung der Krebszellen dienen, gezielt an die Krebszellen zu bringen.

Als Arzneimittel verwendet man vorzugsweise Cytostatika und Metastasehemmer. Beispiele für Cytostatika und Metastasehemmer, die erfindungsgemäß eingesetzt werden können, sind alle derzeit als Cytostatika und Metastasehemmer bekannten Verbindungen. Beispiele hierfür sind Melphalan, Carmustin, Lomustin, Cyclophosphamid, Estramustinphosphat, Ifosfamid, Chlorambucil, Methotrexat, Pegafur, Fluorouracil sowie Antibiotika, die für diese Zwecke eingesetzt werden. Beispiele für Farbstoffe sind Fluoreszenzfarbstoffe, Acridinfarbstoffe, wie Actinomycine, und Rubicin. Diese Verbindungen werden für die Therapie und Diagnostik verwendet.

Das oben erwähnte Kopplungsprodukt aus Jomol und Fluorescein ist ein besonders bevorzugtes erfindungsgemäßes Produkt, da es sowohl für die Diagnose und Therapie als auch zur Markierung mit radioaktivem Jod geeignet ist.

Die Herstellung von markiertem Jomol oder der markierten Jomolderivate, die mit einem Arzneimittel oder einem Farbstoff markiert sind, erfolgt, indem man eine Jomollösung, wie oben bei der Herstellung der Kopplungsprodukte angegeben, oder indem man eine Lösung oder Suspension der Kopplungsprodukte in geeigneter Konzentration mit einer Lösung oder Suspension eines Arzneimittels oder eines Farbstoffs umsetzt. Im allgemeinen werden äquimolare Mengen von Jomol und dem Farbstoff/Arzneimittel/Cytostatikum (beispielsweise Actinomycin C, Sigma A4639®, Sigma, München) in einem polaren Lösungmittel zusammemgebracht. Danach wird eine äquimolare Menge eines Vernetzungsmittels (Glutaraldehyd, Sigma G5882) in an sich bekannter Weise zugesetzt und zur Kopplung der Komponenten verwendet. Die Reaktionstemperatur beträgt 15 bis 40 °C. Anschließend erfolgt eine chromatographische Reinigung.

Die Herstellung der mit einem radioaktiven Marker markierten Produkte erfolgt in an sich bekannter Weise, indem man Jomol oder ein Kopplungsprodukt von Jomol oder eine Lösung mit einer Salzlösung des radioaktiven Markers umsetzt und das radioaktive Produkt in an sich bekannter Weise, beispielsweise durch Säulenchromatographie, gewinnt. Die Markierung mit radioaktiven Strahlern ist in der Literatur beschrieben.

Beispiele für radioaktive Strahler sind die Strahler, die man üblicherweise auf dem Gebiet der Therapie und der Diagnose von malignen Tumoren verwendet, wie beispielsweise $^{99m}$Technetium, $^{111}$Indium, $^{123}$Jod, $^{125}$Jod, $^{130}$Jod, $^{131}$Jod, $^{132}$Jod und $^{224}$Radium. Die Radioaktivität kann beispielsweise 5 µCi bis 200 mCi betragen.

Bei Beladung von Jomol mit einem Radionuclid zeigt der Peak von Jomol mit einem apparenten Molekulargewicht von 3000 in einem FPLC-Elutionsmuster die höchste Menge des Radionuclids. Dasselbe gilt für Jomo-tech, Jomo-In und Jomo-Ra. Bei JomolSn-$^{99m}$Tc, Jomol-DTPA-$^{111}$In und Jomol-CE-$^{224}$Ra wurde nach fünftägiger Lagerung in wäßriger Lösung 0,5 mg/ml bei -20 °C nach der Markierung keine Autoradiolyse festgestellt.

Erfindungsgemäß ist es auch möglich, Gemische aus Jomol und seinen mit einem radioaktiven Strahler, einem Farbstoff oder einem Cytostatikum markierten Derivaten zu verwenden.

Gemäß der vorliegenden Erfindung können Jomol, seine Kopplungsprodukt und die entsprechenden markierten Derivate, wie oben beschrieben, auf Liposomen oder in lipidierter Form verwendet werden.

Liposomen sind kugelförmige Gebilde aus einer oder mehreren Lipiddoppelschichten mit einem Innenraum. Derartige Bläschen lassen sich durch mechanische Feinstverteilung von Phospholipiden, wie zum Beispiel Lecithin, in wäßrigen Medien herstellen.

Erfindungsgemäß werden Liposomen verwendet, die einzelne unilamellare Bläschen (SUV) sind und vorzugsweise aus Phosphatidylcholin : Phosphatidylserin : Cholesterol im molaren Verhältnis 8 : 2 : 10 bestehen und durch Sonication hergestellt werden. Die Lipide, aus denen sie hergestellt werden, sind im Handel erhältlich, beispielsweise von Sigma Products. Die Lipide werden in Ether gelöst, durch Säulenchromatographie gereinigt, unter $N_2$ mit Jomol bzw. seinen Derivaten vermischt, in phosphatgepufferte Kochsalzlösung (PBS), beispielsweise bei pH 7,4, suspendiert und dann beispielsweise 25 Minuten bei +2 °C mit einem pulsierenden Branson-15-Sonicator beschallt (sonicated). Die Sonication wird im allgemeinen unter $N_2$ durchgeführt.

Nach der Sonication werden die Liposomen auf einer Sepharose-4-B-Säule chromatographiert und vorzugsweise die Fraktionen der Population mit Radii unter 300 Å benutzt (C. Huang, Biochemistry 15, 2362 (1969)).

Diese Liposomen werden dann zur Diagnostik in an sich bekannter Weise mit vorzugsweise $^{99m}$Technetium am Jomol markiert.

Um die radioaktive Markierung zu prüfen, wird ein Aliquot der Liposomen auf eine Sepharose-4-B-Säule gegeben und chromatographiert. Man stellt fest, daß die Präparation eine spezifische Aktivität von 99,2% an Jomol gebundene Radioaktivität und 0,8% von freiem Pertechnetat hat.

Die Liposomen können mit Jomol bzw. seinen Derivaten beschickt sein, die mit einem radioaktiven Tracer, mit einem Farbstoff, einem Cytostatikum oder mit Gemischen dieser Verbindungen beschickt sind. Derartige Liposomen sind insbesondere für die Diagnose geeignet.

Gemäß einer erfindungsgemäßen Ausführungsform wird Jomol oder Jomolderivat, gegebenenfalls - wie oben erläutert - beschickt, in einem Lipid dispergiert. Die Dispersion erfolgt, indem man die Substanzen zusammentringt und ebenfalls einer Beschallung unterwirft.

Als Lipide kann man Phosphatidylcholin allein oder mit Phosphatidylserin und Cholesterol zusammen, beispielsweise im molaren Verhältnis 8 : 2 : 10, verwenden.

Wie oben erwähnt, ist bekannt, daß ein Gemisch aus Acetaldehyd und Ethanol eine cancerotoxische Wirkung hat. Überraschenderweise wurde jetzt gefunden, daß Jomol oder seine Derivate eine besonders gute Wirkung entfalten, wenn sie zusammen mit einem Hilfsmittel, welches als "Cocktail" bezeichnet wird, verabreicht werden. Das Hilfsmittel enthält einen Aldehyd der Formel I

RCHO (I)

worin R ein Wasserstoffatom oder eine geradkettige oder verzweigte Kohlenwasserstoffgruppe mit 1 bis 4 Kohlenstoffatomen bedeutet, und gegebenenfalls einen Alkohol der Formel II

$R^1CH_2OH$ (II)

worin $R^1$ die für R gegebene Bedeutung besitzt. Das Hilfsmittel enthält gegebenenfalls übliche Trägerstoffe und/oder Verdünnungsmittel. Es ist besonders bevorzugt, daß das Hilfsmittel zusätzlich zu dem Aldehyd einen Alkohol enthält.

Überraschenderweise zeigte es sich, daß bei gleichzeitiger und bei zeitlich abgestufter Verwendung des Hilfsmittels zusammen mit Jomol oder Jomolderivaten der Wirkungsgrad von Jomol bzw. Jomolderivaten wesentlich verbessert wird.

Die Erfindung betrifft somit auch ein Erzeugnis, welches das oben beschriebene diagnostische Mittel oder Arzneimittel und das oben beschriebene Hilfsmittel enthält.

Das Hilfsmittel in dem erfindungsgemäßen Erzeugnis kann den Aldehyd als solchen in üblichen pharmakologisch verträglichen Trägern und/oder Verdünnungsmitteln enthalten. Besonders bevorzugt ist es, den Aldehyd in wäßriger und/oder alkoholischer Lösung einzusetzen. Erfindungsgemäß ist es dabei besonders bevorzugt, den jeweiligen Aldehyd zusammen mit seinem zugehörigen Alkohol zu verwenden.

Bevorzugte Hilfsmittel setzen direkt oder indirekt frei und/oder enthalten: Formaldehyd/Methanol, Acetaldehyd/Ethanol, n-Propionaldehyd/n-Propanol, iso-Propionaldehyd/iso-Propanol, n-Butyraldehyd/n-Butanol, iso-Butyraldehyd/iso-Butanol, tert.-Butyraldehyd/tert.-Butanol, n-Valeraldehyd/n-Pentanol oder Gemische dieser Verbindungen.

Das Stoffpaar Acetaldehyd/Ethanol ist in der erforderlichen Konzentration und Menge praktisch ungiftig; man kann es in geeignet hohen Dosen verabreichen. Daraus resultiert die Möglichkeit einer Dauerbehandlung, auch in Kombination mit einer Strahlenbehandlung. Das immunbiologische System wird positiv beeinflußt, und eine Kombination mit anderen Medikamenten sowie mit chirurgischen und radiologischen Maßnahmen ist möglich.

Außer dieser Mischung Ethanol/Acetaldehyd sind grundsätzlich auch andere analoge Mischungen der oben genannten Art möglich. Methanol wird im menschlichen Körper wesentlich langsamer abgebaut als Ethanol, Propanol um den Faktor 2 schneller als Ethanol. Das Mittel kann jeweils nur einen bestimmten ausgewählten Aldehyd wie auch Aldehydmischungen enthalten. Die Anwendung der Aldehyde ist nicht zwingend mit der Anwesenheit der entsprechenden Alkohole gekoppelt. Auch wäßrige Lösungen der Aldehyde können eingesetzt werden. Anstelle der freien Aldehyde können erfindungsgemäß auch solche Aldehydderivate zum Einsatz kommen, die im Stoffwechsel des mit dem erfindungsgemäßen pharmazeutischen Mittel Behandelten den freien Aldehyd bilden. Geeignete Aldehydderivate sind beispielsweise die Acetale oder Halbacetale oder Kondensationsprodukte, die ebenfalls als solche oder in gelöster Form (Wasser oder Alkohole) wie auch in Mischungen mit den Aldehyden und/oder Alkoholen Verwendung finden können.

In einer bevorzugten weiteren erfindungsgemäßen Ausführungsform enthält das Hilfsmittel geringe Mengen (weniger als 0,05 Gew.-%) an Peroxiden, wobei insbesondere die hier stofflich verwandten Peroxide in Betracht kommen, insbesondere $H_2O_2$ und/oder das Aldehydpercxid bzw. Hydroxyhydroperoxid sowie das Peroxid der zugehörigen Carbonsäure. Durch den Gehalt an Peroxiden wird die antitumorale Wirkung noch weiter verbessert.

Die Konzentration des Aldehyds im erfindungsgemäßen Präparat ist einerseits durch dessen Verträglichkeit und andererseits durch die zu verabreichende Dosis bestimmt. Für das Paar Ethanol/Acetaldeyd ist eine Acetaldehydkonzentration im Alkohol unter 2 $\times$ $10^{-4}$ Mol/Liter häufig in der Wirkungsweise unbefriedigend langsam. Die Wirkung steigt mit steigender Aldehydkonzentration und ist nach oben hin in der Regel durch möglicherweise eintretende Unverträglichkeit des Acetaldehyds im Einzelfall begrenzt. In der Praxis bewährt haben sich beispielsweise Ethanol-Aldehyd-Lösungen mit 5 x $10^{-2}$ Mol bis 1 Mol Acetaldehyd pro Liter Ethanol, wobei diese Mischungen in einer Dosis von beispielsweise 10 bis 150 $cm^3$ pro Tag Verwendung finden können.

Es ist bevorzugt, daß das Hilfsmittel 10 bis 60 g Aldehyd pro 1000 g Alkohol, besonders bevorzugt 40 g Aldehyd pro 1000 g Alkohol, enthält. Im allgemeinen wird das Hilfsmittel für die Verabreichung mit Wasser verdünnt. Die alkoholische Lösung kann mit Wasser beliebig verdünnt werden. Beispielsweise kann man ein Volumen der alkoholischen Lösung mit 1 bis 10 Volumen, vorzugsweise 2 bis 5 Volumen, Wasser verdünnen.

Das Hilfsmittel wird bevorzugt oral in Form der wäßrigen Lösung verabreicht und vom Patienten getrunken. Das Hilfsmittel kann jedoch auch parenteral verabreicht werden.

In dem erfindungsgemäßen Erzeugnis bzw. Kit können die beiden Bestandteile jeweils auf unterschiedliche Weise kombiniert sein. Das Hilfsmittel kann in einer für die orale Verabreichung und/oder für die parenterale Verabreichung, zum Beispiel durch Infusion, geeigneten Form vorliegen. Die Zubereitung von Infusionslösungen ist dem Fachmann geläufig und kann in an sich bekannter Weise erfolgen. Beispielsweise kann das Hilfsmittel in Form von Trinkampullen vorliegen, oder es kann in Form von Trinkampullen, die mit Wasser verdünnt werden, vorliegen.

Für die Diagnose ist es bevorzugt, zuerst das Hilfsmittel oral als wäßrige Lösung und ca. 20 bis 30 Minuten später das Diagnostikum parenteral, bevorzugt intravenös, zu verabreichen. Für die Therapie ist es ebenfalls bevorzugt, zuerst das Hilfsmittel oral, wie oben beschrieben, zu verabreichen und dann das Arzneimittel 20 bis 30 Minuten später zu verabreichen. Das Arzneimittel wird durch Inhalation, intravenös oder intratumoral verabreicht.

Im allgemeinen beträgt die erforderliche Jomoldosis sowohl für die Therapie als auch für die Diagnose 10 bis 500 $\mu$g, wobei für die Diagnose vorteilhafterweise 10 bis 50 $\mu$g als einmalige Dosis und für die Therapie 10 bis 500 $\mu$g pro Tag verwendet werden. Die Dauer der Behandlung hängt vom Einzelfall ab. Es ist bevorzugt, eine Einzeldosis in einer Flasche steril aufzubewahren. Zur Verabreichung kann diese beispielsweise mit Kochsalzlösung für die Injektion verdünnt oder in eine Inhalationsvorrichtung gegeben werden.

In dem Kit liegen die Bestandteile vorzugsweise in getrennten Behältern vor, wobei beispielsweise das Hilfsmittel in einer Trinkampulle oder einer Schraubdeckelflasche vorhanden sein kann und das Jomol oder eines seiner Derivate in einer fest abgedichteten Flasche oder Ampulle vorliegt.

Jomol und die Jomolderivate werden im Lichtschutzkarton unter Stickstoff bei -40 bis -10 °C aufbewahrt. Ausnahme sind die Jomolpräparate auf Liposomen oder lipidiert, die bei +4 °C bis +10 °C aufbewahrt werden. Das Kit kann einen dritten Behälter enthalten, in dem beispielsweise ein Lösungsmittel vorgesehen ist, das kurz vor Gebrauch zu dem Jomol bzw. einem seiner Derivate zugegeben wird. Lösungsmittel sind alle solche, die pharmazeutisch annehmbar sind und Jomol bzw. seine Derivate lösen, zum Beispiel physiologische Kochsalzlösung, PBS und andere.

Es ist jedoch bevorzugt, das Hilfsmittel herzustellen, indem man einen Alkohol der Formel II

$R^1CH_2OH$ (II)

worin $R^1$ ein Wasserstoffatom oder eine geradkettige oder verzweigte Kohlenwasserstoffgruppe mit 1 bis 4 Kohlenstoffatomen bedeutet, mit energiereicher Bestrahlung unter Zutritt von Sauerstoff zu bestrahlen.

Als energiereiche Bestrahlung kann beispielsweise $\gamma$-, UV-, Röntgen- oder Elektronenbestrahlung eingesetzt werden. Es können dabei die ausgewählten Alkohole als solche, aber auch Alkohol-Wasser-Mischungen Verwendung finden, wobei als Ausgangsmaterial hochkonzentrierte Alkohol-Wasser-Mischungen besonders bevorzugt sein können. Die Bestrahlung erfolgt unter Zutritt von Sauerstoff, bevorzugt unter Luftzutritt.

Ein für die Praxis besonders wichtiges und wirksames Antitumormittel läßt sich beispielsweise dadurch herstellen, daß man 96%iges Ethanol in Gegenwart von Sauerstoff energiereicher Bestrahlung der genannten Art aussetzt, bis sich die gewünschte Menge an Acetaldehyd gebildet hat. Die Lösung enthält dann im wesentlichen neben viel Ethanol den Acetaldehyd zusammen mit Peroxiden, wie $H_2O_2$ oder Acetoperoxid, oder Spuren von Peressigsäure sowie Essigsäure. Die zuletzt genannten Substanzen verbessern die Wirkung der mit Jomol bzw. seinen Derivaten beschickten Liposomen wesentlich. Überraschenderweise zeigte sich, daß eine für Ethanol/ Aldehyd typische Vermehrung der Makrophagen, T-Zellen, T-Helferzellen

und Killerzellen gefunden wurde und dieser Effekt die Wirkungsrichtung von Jomol bzw. seinen Derivaten von einer vorwiegenden B-Zellstimulation, wie sie bekannt ist, auf eine Vermehrung der Makrophagen, T-Zellen, T-Helfer- und Natural-Killerzellen umlenkt, so daß bis dahin unbekannte große Zahlen dieser Zellen im Menschen erzeugt werden. Dies wird in der vorgenannten Liteaturstelle von U. Ehrenfeld nicht beschrieben, es lag auch nicht nahe.

Jomol und seine Derivate (unter "Jomolderivaten" werden in der vorliegenden Anmeldung alle Kopplungsprodukte und markierten Produkte verstanden) dringen überraschenderweise leicht durch die Zellwände hindurch, insbesondere durch die Wände von Lungenalveolen, Lymph- und Blutkapillaren, und reichern sich an Krebszellen an, wo sie leicht nachgewiesen werden können. Dadurch werden eine neue Diagnostik und eine neuartige Behandlungsweise von malignen Tumoren ermöglicht, da das mit einem radioaktiven Marker markierte Jomol bzw. solche Jomolderivate unmittelbar zum malignen Tumor gelangen und dort zur Erkennung und Behandlung des Tumors die höchstmögliche Wirkung erbringen.

Das erfindungsgemäße Diagnostikmittel kann zur Diagnostik von malignen Tumoren in vivo und in vitro verwendet werden. Die malignen Tumoren können außerhalb des Körpers diagnostiziert werden. Beispielsweise kann der Chirurg bei einer Operation malignes Gewebe entfernen und dann das lebende Gewebe außerhalb des Organismus inkubieren, so daß im flachen Anschnitt des Gewebes, zum Beispiel bei Farbmarkierung mit Fluoresceinisothiocyanat im UV-Licht malignes Gewebe aufleuchtet und von dunklem gesundem Gewebe sofort unterschieden werden kann.

Bei Applikation des Cocktails per infusionem und anschließender intravenöser Gabe von Jomo-Color wird im UV-Licht intraoperativ Tumormaterial sichtbar. Bei einer lymphogenen Aussaat von Tumormaterial werden die mit Jomol-Fluoresceinisothiocyanat markierten Zellen des Tumors rasch phagozytiert. Mit dem erfindungsgemäßen Mittel lassen sich Tumorgrößenordnungen von 100 bis 10.000 Zellen exakt kennzeichnen; bei zusätzlicher Sauerstoffgabe ist das Mittel in der Lage, anhand der für die Diagnostik eingebrachten minimalen radioaktiven Strahlung eine floride Entzündung infolge Strahlenzerstörung von Tumorgewebe zu erzeugen.

Gemäß der vorliegenden Erfindung ist es somit erstmals möglich, maligne Tumoren auf einfache Weise, ohne daß der Patient belastet wird, bei Säugetieren, insbesondere bei Menschen, zu diagnostizieren und auch zu heilen. Erfindungsgemäß ist es möglich, die Tumoren gezielt zu bestrahlen, fast ohne daß Nachbargewebe geschädigt wird, und weiterhin kann man Medikamente, zum Beispiel die Cytostatika oder Immunmodulatoren, erfindungsgemäß an den Ort bringen, wo sie tatsächlich wirken sollen.

Wenn Jomol bzw. eines seiner Derivate mit einem radioaktiven Strahler markiert ist, erfolgt die Sichtbarmachung der radioaktiven Verteilung unter Verwendung einer externen Gamma-Kamera. Dargestellt werden mit der externen Gamma-Kamera verschiedene Melanosarkome, Plattenepithelkarzinome und Adenokarzinome der weiblichen Brust. Die Tests sind reproduzierbar.

Fluoresceinisothiocyanat oder $^{99m}$Technetium bleiben an der Krebszellenwand an Jomol bzw. dessen Derivat gebunden und sind dort fluoreszenzmikroskopisch bzw. autoradiographisch nachweisbar. Beim Abbilden mit der Gamma-Kamera ist im Modell mit der SD-Ratte an Walker-Carcinosarkom 256 die Anbindungsrate der erfindungsgemäß beanspruchten Substanz unter gleichzeitigem Einsatz der Kombination (Cocktail) aus Ethanol/Acetaldehyd/Wasser 2,5- bis 8mal höher als am gesunden Gewebe (im Seitenvergleich). Beim Menschen ist das Anbindeverhältnis höher. Da Jomol auf dem Blutwege im Körper verteilt wird und über Nieren, Lebe und Lunge in Urin, Galle und abgeatmete Luft ausgeschieden wird, sind bei entsprechender Markierung des Jomol bzw. seiner Derivate mit radioaktiven Isotopen bei Darstellung mittels Gamma-Kamera außer Krebs und seinen Metastasen in Weichteilen und Knochen auch Funktions- bzw. Clearance-Untersuchungen bei diesen Passagen durchführbar.

Pharmakologische Untersuchungen

1. Toxizität

Jomol und seine Derivate sind praktisch untoxisch. Im Screening nach Robert A. Turner (ausgeführt durch die Firma Pharmatox, Sehnde, FRG) fanden sich für Jomol, Jomo-tech, Jomo-In und Jomo-Color bei einer rechnerischen Überdosis der Humantagesdosis vom 4000- bis 5000fachen, d.h. in einer Menge von 2 mg/kg Körpergewicht, an SPF-Mäusen keine Abweichungen von Normalbefunden. Die Verträglichkeit erscheint ausgezeichnet unerwünschte Nebenwirkungen traten nicht auf.

2. Bindung von radioaktiv markierten Jomolderivaten an maligne und benigne Zellen in vitro

Um zu zeigen, daß Jomol und seine Derivate an Karzinomzellen besonders leicht gebunden werden,

wurden Vergleichsversuche durchgeführt. Es wurde das Bindungsverhalten der radioaktiven Komponenten Jomol-DTPA-$^{111}$In, Jomol-CE-$^{224}$Ra und Jomol-Sn-$^{99m}$Tc an Karzinomzellen und an gesunden Zellen in vitro geprüft. Als Karzinomzellen wurden embryonale Mäusezellen F9 und als gesunde Zellen Mäusefibroblasten L929 verwendet.

In einem weiteren Versuch wurde gezeigt, daß die Anbindung besonders ausgeprägt ist, wenn vor der Verabreichung der radioaktiven Komponenten der erfindungsgemäße Cocktail verabreicht wird.

Für die Bewertung der Bindungsergebnisse ist es erheblich, daß das Volumen einer durchschnittlichen gesunden Fibroblastenzelle L929 etwa dreimal so groß ist wie das einer maligen F9-Zelle, die Oberfläche demzufolge zweimal so groß. Zudem ist das Verhältnis der Zellwand- und Adhäsionsmatrix zur Zelle bei den gesunden L929-Zellen deutlich größer als bei den malignen F9-Zellen.

Das Ergebnis der Bindungsexperimente ist in der folgenden Tabelle I zusammengefaßt. Summarisch kann festgestellt werden, daß die embryonalen Mäusekarzinomzellen F9 Jomol-DTPA-$^{111}$In, Jomol-CE-$^{224}$Ra und Jomol-Sn-$^{99m}$Tc um einen Faktor von ca. 30 bis 60 besser binden. Es ist eine tendenzielle Steigerung des Bindungsverhältnisses durch Zusatz des Cocktails aus Alkohol und Acetaldehyd erkennbar.

Tabelle I

Verhältnis der spezifischen Zellmarkierung (cpm/$10^6$ ausgesäte Zellen, normiert) von embryonalen Mäusekarzinomzellen F9 zu Mäusefibroblasten L929 (F9/L929)

| Bindungsmedium | Jomol-DTPA-$^{111}$In | | Jomol-CE-$^{224}$Ra | Jomol-Sn-$^{99m}$Tc |
|---|---|---|---|---|
| DMEM, 10% FCS | 34,2 | 28,6 | 30,6 | 12,6 |
| DMEM, 10% FCS 500 µg/ml EtOH | 42,0 | 33,6 | 49,2 | 33,6 |
| DMEM, 10% FCS 500 µg/ml EtOH 2 mM Acetaldehyd | 56,4 | 28,0 | 62,4 | 33,6 |
| DMEM, 10% FCS 500 µg/ml EtOH 4 mM Acetaldehyd | 150,0 | 48,6 | 44,4 | 26,4 |

DMEM, 10% FCS = Dulbecco's Modified Eagle Medium mit 10% fötalem Kälberserum

EtOH = Ethanol

L929 Mäusefibroblasten (ATCC CCL1, NCTC clone 929, Connective tissue, mouse)

F9 embryonale Mäuse-CA-Zellen, H2-negativ, Linie 129, Labor F. Jakob, Institut Pasteur, Paris

3. Bindung des radioaktiv markierten Jomols an malignes Tumorgewebe in vivo

a) Bindung an Walker-Carcinosarkom 256 in der Sprague-Dawley-Ratte

Je drei weiblichen Sprague-Dawley-Ratten wurde 4 Tage nach subkutaner Transplantation von Walker-Carcinosarkom 256 0,8 ml einer Jomol-DTPA-$^{111}$In-Lösung bzw. einer Jomol-Sn-$^{99m}$Tc-Lösung appliziert. Die erste Lösung enthielt ca. 80 nmol/ml Jomol-DTPA-$^{111}$In in einer spezifischen Markierung von ca. 7 Ci/mmol. Die zweite Lösung enthielt ca. 68 nmol/ml Jomol-Sn-$^{99m}$Tc in einer spezifischen Markierung von ca. 25 Ci/mmol. Allen Tieren wurde 30 Minuten vor i.v.-Gabe des markierten Jomols 1 ml Cocktail oral verabreicht (Cocktail: 22,5 ml physiologische Kochsalzlösung, 2,4 ml Ethanol, 0,26 ml Acetaldehyd). Die Tiere wurden 24 Stunden p.a. in Chloralhydrat-Narkose aus dorsaler Sicht szintigraphiert.

Der Tumor war bei allen Tieren dertlich abgrenzbar. Die Tumormarkierung, angegeben als Quotient der spezifischen Anreicherung an Tumor und Muskelgewebe, ergab sich für Jomol-DTPA-$^{111}$In zwischen 4:1 bis 8:1, für Jomol-Sn-$^{99m}$Tc zwischen 4:1 bis 6:1. In weiteren Folgeexperimenten wurden recht häufig Bindungsverhältnisse bei 8:1, in Einzelfällen bis zu 36:1, gefunden.

b) Verteilung von Jomol-Sn-$^{99m}$Tc in der tumortragenden Sprague-Dawley-Ratte

Fünf weiblichen Sprague-Dawley-Ratten wurde 4 Tage nach subkutaner Transplantation von Walker-Carcinosarkom 256 in die rechte Seitenflanke jeweils 0,5 ml einer Jomol-Sn-$^{99m}$Tc-Lösung in die Schwanzvene injiziert. Die Lösung enthielt 25 mmol/ml Jomol-Sn-$^{99m}$Tc in einer spezifischen Markierung von 25 Ci/mmol. 30 Minuten vor Applikation wurde den Tieren 2,5 ml Cocktail oral verabreicht (Cocktail: 22,5 ml physiologische Kochsalzlösung, 2,4 ml Ethanol, 0,26 ml Acetaldehyd).

Zwei der Tiere ergaben im Szintigramm ein Anbindungsverhältnis (spezifische Anreicherung im Tumor zu spezifischer Anreicherung im Muskelgewebe) von 6:1 bei einem mittleren Tumorgewicht von ca. 1,5 g. Szintigraphisch konnte der Tumor bereits 30 Minuten nach Applikation abgegrenzt werden. Extrapoliert auf die Verhältnisse beim Menschen kann erwartet werden, daß hier eine Tumorszintigraphie bereits 3 Stunden nach Applikation möglich sein wird. Zur Ermittlung der Verteilung in den Organen wurden die Tiere eine Stunde nach Injektion dekapitiert und weitgehend entblutet. Die in Tabelle II aufgeführten Organe wurden entnommen und gewogen; die enthaltene Radioaktivität wurde in einem Gamma-Counter gemessen.

Die folgende Tabelle II zeigt die Verteilung der Radioaktivität eine Stunde nach Applikation.

Ein nicht unbeträchtlicher Anteil der Radioaktivität wurde innerhalb einer Stunde renal ausgeschieden; die geschätzte Clearance beträgt 0,5 ml/min.

Tabelle II

In-vivo-Verteilung von $^{99m}$Tc-Jomol in der tumortragenden Ratte eine Stunde nach intravenöser Applikation (n = 5, Sprague-Dawley, Walker-Carcinosarkom 256)

| Organ | % d. Dosis/g |
|---|---|
| Blut | 0,46 ± 0,01 |
| Leber | 0,17 ± 0,05 |
| Milz | 0,09 ± 0,01 |
| Magen | 0,14 ± 0,02 |
| Nieren | 3,34 ± 0,29 |
| Herz | 0,16 ± 0,02 |
| Lunge | 0,24 ± 0,02 |
| Muskel | 0,05 ± 0,01 |
| Thymus | 0,11 ± 0,04 |
| Femur | 0,09 ± 0,02 |
| Tumor | 0,32 ± 0,05 |

c) Am Menschen wurden verschiedene maligne Tumoren dargestellt. Mit Hilfe einer Gamma-Kamera

wurden 3 Stunden nach intravenöser Gabe von Jomo-tech (20 mCi $^{99m}$Technetium) bzw. Jomo-In (2 bis 3 mCi $^{111}$Indium) (in den meisten Fällen wurde eine halbe Stunde zuvor eine Dosis Cocktail - 24 ml Ethanol, 1 ml Acetaldehyd, 225 ml Wasser - oral verabreicht) die Tumoren abgebildet. Am Ort der Tumorabbildung wurde mit anderen Untersuchungen (zum Beispiel histologisch) der Tumor verifiziert. Das Verhältnis Tumorareal/Nichttumorareal lag hinsichtlich der durch die Gamma-Kamera detektierten Strahlung über 10:1, in der Leber über 8:1.

4. Therapeutische Wirkung von Jomol-CE-$^{224}$Ra

22 Sprague-Dawley-Ratten wurde 3 Tage nach subkutaner Transplantation von Walker-Carcinosarkom 256 in den Oberschenkel jeweils 1 ml einer Lösung mt Jomol-CE-$^{224}$Ra in die Schwanzvene injiziert (0,8 Ci/mmol, 13 nmol/ml). 30 Minuten vor Applikation wurde den Tieren 2,5 ml Cocktail oral verabreicht (Cocktail: 22,5 ml physiologische Kochsalzlösung, 2,4 ml Ethanol, 0,26 ml Acetaldehyd).

Die Tiere wurden je nach Fragestellurg ab dem 3. Tag nach Applikation entweder getötet, oder sie starben nach dem 8. bis 10. Tag. Die Tumoren wurden entnommen und gewogen. Die Korrelation lg Tumorgewicht/Zeit nach Applikation wurde einer Regressionsanalyse unterzogen und mit einer entsprechenden Korrelation unbehandelter Tiere verglichen.

Die Tumorentwicklung in der exponentiellen Wachstumsphase läßt sich nach einmaliger Gabe von Jomol-CE-$^{224}$Ra mit einer Geraden der Form lg y = 0,19 × -0,66 beschreiben.

Die Entwicklung unbehandelter Tumoren (entsprechend "Tumor-Steckbrief", DKFZ Heidelberg, Deutschland) läßt sich mit einer Geraden der Form lg y = 0,28 × -1,28 beschreiben. Das Wachstum in der behandelten Gruppe hat sich verglichen damit um ca. 30% reduziert.

5. Verhalten von $^{131}$Jod-Jomol, Autoradiographie von Tumorzellen eines Walker-Carcinosarcoms 256 auf einer männlichen Sprague-Dawley-Ratte 4 Tage nach intravenöser Applikation

| | |
|---|---|
| Präparation: | 281 µCi $^{131}$Jod/µmol Jomol |
| Eingesetzte Dosis: | 30 µCi $^{131}$Jod, gebunden an Jomol |
| Molares Verhältnis: | $^{131}$J-Jomol/Jomol unmarkiert wie 1:1,3 x $10^4$ |
| Versuchsdauer (Präparation bis Ende der Autoradiographie): | 8 Tage (davon Autoradiographie 3 Tage) |
| Gewicht der SD-Ratte: | ca. 200 g |
| Zellzahl der SD-Ratte: | ca. 6 x $10^{11}$ (geschätzt für 200 g Körpergewicht) |

Bei statistischer Verteilung sind nach i.v.-Applikation 7 Moleküle $^{131}$J-Jomol pro Zelle der SD-Ratte zu erwarten. Nach 4 Tagen ist bei einer Ausscheidung im Harn von ca. 1 nmol Jod bei der Ratte pro Tag von einer gegebenen Dosis von 14 pmol $^{131}$J weniger als $10^{-8}$ dieser Dosis vorhanden. $10^{-8}$ von 14 pmol $^{131}$J ergeben nach Ablauf einer Halbwertszeit seit Präparation 7 Atome $^{131}$Jod pro $10^{11}$ Zellen. Pro Zelle des abgebildeten Schnittes des Walker-Carcinosarkoms 256 der SD-Ratte findet sich autoradiographisch >1 Zerfall von $^{131}$Jod bei einer Autoradiographiedauer von 3 Tagen.

Bei einem molaren Verhältnis, wie es in der applizierten Dosis vorlag, muß von >5 × $10^4$ Bindungsstellen für Jomol pro Zelle ausgegangen werden. Eine Abreicherung von tumorgebundenem $^{131}$J-Jomol ist innerhalb der Versuchsdauer nicht beobachtbar.

6. Immunstimulatorische Wirkung von Cocktail und Jomol (bzw. Jomol-Aer) am Menschen

Wie bereits festgestellt, führt Jomol besonders zusammen mit dem Cocktail (erfindungsgemäße Kombination) zur Steigerung der zelligen Immunabwehr beim Menschen.

Beim Einsatz des Cocktails aus 50 g 96%igem Ethanol, 2 g reinstem Acetaldehyd und 450 g Wasser, oral, und Inhalation von Jomol auf Liposomen aus Phosphatidylcholin, Phosphatidylserin und Cholesterol im molaren Verhältnis von 8:2:10 in physiologischer Kochsalzlösung zeigt sich für eine Dosis von 30 bis 60 µg Jomol (auf 5 mg Liposomen in 5 ml physiologischer wäßriger Kochsalzlösung) ein Wirkungsoptimum.

An einem Fallbeispiel wird im folgenden die Wirkung dargestellt. Hierbei wurde Jomol-Aer mit einem Siemens-Mikroinhalator vernebelt und inhaliert. Vorab wurde Cocktail verabreicht.

Blutbildauswertungen:

Bei vorher vorliegenden Normwerten, zum Beispiel 7400 Leukozyten/µl,Monozyten, T- und B-Lymphozyten im Normbereich, steigen die Werte 24 Stunden nach Applikation auf beispielsweise 24.800 Leukozyten/µl. Die Lymphozyten erreichen folgende Werte:

| | pro µl |
|---|---|
| T-Lymphozyten, gesamt: | |
| über E-Rosetten | 11.840 |
| über monoklonale Antikörper | 12.640 |
| T-Helferzellen über monoklonale Antikörper | 8.960 |
| T-Suppressorzellen über monoklonale Antikörper und zytotoxische monoklonale Antikörper | 2.560 |
| T-helfer-/T-suppressor-cytotoxische = 3,5 | |
| B-Lymphozyten Ig-oberflächenpositiv | 1.600 |
| Monozyten über monoklonale Antikörper | 2.400 |
| Lymphozyten $Ia^+$ (aktivierte B- und T-Lymphozyten und einige Monozyten) über monoklonale Antikörper | 1.120 |
| OKT 11 | 13.760 |
| Leu 7 | 2.240 |

Antwort der Lymphozyten auf Mitogene (Ergebnisse der Isotopenmessung, cpm):

| | cpm ± e.s. |
|---|---|
| PHA: | 86.578 ± 4.768 |
| ConA: | 87.536 ± 6.755 |
| ohne Mitogen (Kontrollen): | 309 ± 118 |

Allgemein ist festzustellen, daß die Steigerung der zelligen Immunabwehr beim Menschen, wie sie am

Beispiel der Monozyten über Monate und Jahre verfolgt wurde, nicht erschöpfbar ist. Von den üblichen geringen physiologischen Schwankungen abgesehen, ist durch den Einsatz des beanspruchten erfindungsgemäßen Mittels, genannt "Jomol", stets eine ausgeprägte Steigerung der Granulozytenzahl pro µl, bei jüngeren Menschen um mehr als 3.000/µl, bei alten Menschen um ca. 1.500/µl, sowie eine Steigerung der Monozytenzahl vom Normwert 0 bis 140/µl auf ca. 2.000/µl Blut (bei alten Menschen auf ca. 900 bis 1.000/µl) erreicht worden.

7. Zur diagnostischen/therapeutischen Wirkung von Jomo-Color (Jomol-Fluoresceinisothiocyanat) (Jomol-Fitc)

Aus Versuchen mit Fitc-markierten Lymphozyten ist bekannt, daß diese in vivo in wenigen Stunden phagozytiert werden. Segmentkernige Leukozyten (Granulozyten) besitzen proteolytische Enzyme und Lysozym. Das Lysozym dient zur Spaltung von Murein in Bakterien.

Überraschenderweise wurde nun gefunden, daß das durch Fitc "verfremdete" Mureinbruchstück Jomol (bei Cocktail-Gabe verstärkt) an Tumorzellen gebunden im Bereich der proteasenveränderten Zellwandmatrix Granulozyteninvasionen auslöst. Wenn Walker-Carcinosarcom-256-Ascites mit Jomol-Fitc, Ethanol/Acetaldehyd und p-Hydroxyphenyl-α-ketopropionsäureantagonisten (die durch Konkurrenz an deren Rezeptor wirken) oder tyrosinanaloge Moleküle und Monoaminooxidasehemmer auf Sprague-Dawley-Ratten behandelt wird, so findet man nach 24 Stunden, beispielsweise nach Einsatz von Jomol-Fitc, Ethanol/Acetaldehyd und Cotrimoxazol im Punktat des Ascites eine sehr große Zahl von Leukozyten und eine subtotale regressive Veränderung der Ascitestumorzellen. Die Kontrollen zeigen vitale Tumorzellen und spärlich Leukozyten. Beispiele für die oben genannten tyrosinanalogen und Tyrosinabbauproduktantagonisten sind Sulfamethoxazol und Isoniazid mit dem Monoaminooxidasehemmer Tranylcypromin.

Dieser Effekt ist wie die selektiv auftretende Fluoreszenz der Krebszellwände (fluoreszenz-)mikroskopisch bereits 10 bis 15 Minuten nach i.v.-Applikation von Jomo-Color festzustellen. In sieben Versuchen mit insgesamt 131 Sprague-Dawley-Ratten (mit jeweils 2 bis 3 g Walker-Carcinosarcom-Tumor) wurde nach i.v.-Gabe von ca. 30 µg Jomol-Fitc je Tier an ca. einem Drittel der Tiere 24 bis 48 Stunden p.a. eine subtotale bis totale Kolliquation des Tumors gefunden. Bei den übrigen Tieren war dieser Vorgang klinisch nicht zu beobachten.

Die folgenden Beispiele erläutern die Erfindung.

**Beispiel 1**

Herstellung von Jomol

Nocardia opaca (ATCC 21 953) werden auf DST-Platten (Oxoid, Wesel, FRG) oder Traubenzuckeragar, 2% (Merck, Darmstadt, FRG) in Plattentechnik auf drei bis vier Platten 5 bis 8 Tage kultiviert. Nach dam Abernten werden die Bakterien auf ca. 100 ml normale Nährbouillon verimpft und über Nacht bei 30 bis 37° C bebrütet.

Das Ernten der Nocardia-Bakterien erfolgt vorzugsweise durch Abzentrifugieren, beispielsweise bei 4000 Umdrehungen/Minute, 5 bis 10 Minuten lang in der "Roto-Silenta-K"-Hettich-Kühlzentrifuge (oder anderen geeigneten) mit einem Rotor von 40 cm Durchmesser. Der erhaltene Bodensatz (ca. 5 g Bakterien) wird in 100 ml beispielsweise 0,01 M Tris/HCl-Puffer (pH 7,4), vorzugsweise mit EDTA-$Na_2$ (0,06 %) suspendiert (Waschvorgang), wie oben angegeben, abzentrifugiert und in ca. 100 ml 0,01 M Trs/HCl-Puffer von pH 7,4 mit vorzugsweise 0,06 % EDTA-$Na_2$ und mit 10 % Glucose suspendiert. Nach ca. 30 Minuten werden 10 mg Lysozym (Lysozyme, Sigma L-6876, Sigma, München, FRG) und vorzugsweise zur Herabsetzung der Viskosität 3 mg Desoxyribonuclease (Deoxyribonuclease, Sigma D-0876) zugesetzt. Diese Phase mit Glucose und Fermenten dauert 2 Stunden und findet bei 30 bis 37° C statt. Dann kann, wie oben angegeben, abzentrifugiert werden. Der Bodensatz kann in 100 ml 0,01 M Tris-HCl-Puffer mit 0,06 % EDTA-$Na_2$ resuspendiert werden und erneut abzentrifugiert und im selben Medium resuspendiert werden. Dieser Waschvorgang kann unterbleiben, aber durch ihn wird mehr Wandmaterial der Bakterien und Nährmedium aus dem abschließend verwendeten Überstand entfernt.

Danach werden die Zellen zerstört. Dies kann mit allen geeigneten Methoden geschehen, bevorzugt wird Ultraschall über 1 Minute, zum Beispiel mit dem "Sonicator cell disruptor" von Kontron Ultrasonics, Modell W 185 F, 15 mm Arbeitsende, Stufe 3, Skala bei 50.

Danach wird bei 4 bis 6° C in der Kühlzentrifuge (wie oben beschrieben) bei 4000 Upm 10 bis 15 Minuten lang zentrifugiert.

Das UV-Spektrum des Überstands ist in Figur 1 dargestellt.

Der Überstand wird vorzugsweise in fünf Teile geteilt und auf fünf vorbereitete Sephadex-G-75-Säulen zu je einem Teil gegeben. Die Säulen sind 80 cm lang, Innendurchmesser 2,5 cm, die Sephadex-Füllungshöhe beträgt 60 cm (UV-Detektion bei 214 nm). Bei Vewendung einer Säule werden die übrigen Portionen bei -25 ° C bis zur Verwendung eingefroren. Die Fraktion hat ca. 4500 bis 900 Dalton. Sie tritt beim Eluieren zum Beispiel mit 0,01 M Tris-HCl-Puffer (pH 7,4), mit 0,06 % EDTA-$Na_2$ bei ca. 900 bis 980 ml durch, bei einer Tropfgeschwindigkeit von etwa 2 Tropfen pro Sekunde (über Nacht) nach ca. 12 Stunden in 1 bis 2 Stunden. Diese Fraktion enthält die aktive Fraktion, d.h., das gewünschte Produkt 2b*. Es folgt eine Lyophilisation. Danach wird vorzugsweise 1 Stunde bei 80 ° C inkubiert.

Wenn nicht lyophilisiert wird, dann sind die verdünnten Lösungen bei -20 ° C, besser bei -40 ° C bzw. -80 ° C, eingefroren aufzubewahren.

Jomol wird durch Umsetzung von Fraktion 2b* (angenommenes mittleres Molekulargewicht: 4000) mit Acetaldehyd im molaren Verhältnis 2b*:Acetaldehyd wie 1:2 erhalten. Danach muß erneut lyophilisiert werden, gegebenenfalls (siehe oben)eingefroren.

Die Ausbeute für ca. 5 g Bakterien beträgt ca. 2 mg reines Produkt, d.h. Jomol. Das Produkt ist sehr hygroskopisch, Wägungen unter Feuchtigkeitszutritt (Luft) erlauben keine exakten Substanzmengenangaben, deshalb im vorher gewogenen verschlossenen Gefäß nach der Gefriertrocknung wiegen und gegebenenfalls mit den üblichen Methoden eine Wassergehaltsbestimmung des Lyophilisats durchführen. Das Jomol zeigt das in Figur 2 dargestellte UV-Spektrum. Es besitzt die folgenden Eigenschaften:

Absorptionsmaximum im UV-Spektrum bei 282,5 nm. Es wurde ein Spektralphotometer UVIKON 860 (1984), Kontron, Zürich, Schweiz, verwendet und die Substanz wurde in Wasser gelöst, Referenz Wasser 1 cm Quarzküvetten;
apparente Molekulargewichte bei 4500, 3000 und 900;
je nach Restwassergehalt elfenbeinfarbig amorph-kristallin-flockig;
pH-Wert von 5,5 (1 mg der Verbindung, gelöst in 1 ml Wasser);
schwach positive, rosa Ninhydrin-Reaktion;
anfärbbar mit Orcin Reagens;
Peptidanteil: ca. 50 %, bezogen auf amorphe Substanz (Proteintest MicroBioRad nach L. Thomas, J.Clin.Chem. Clin.Biochem., Bd. 19, 1981, Seite 203-208, Fa. BioRad, München; Coomassie Brilliant Blue G 250 ®);
folgende $R_f$-Werte (Lösungsmittelangabe Volumen:Volumen)

| | | |
|---|---|---|
| $R_f$ = 0,20 | (Butanol:Eisessig:Wasser | 4:1:1) |
| $R_f$ = 0,45 | (Benzol:Eisessig:Wasser | 2:1:2) |
| $R_f$ = 0,685 | (Methanol:Eisessig:Wasser | 4:1:1) |
| keine Wanderung | (Chloroform:Methanol) | 96:4) |
| $R_f$ = 0,54 | (Chloroform:Methanol) | 1:1); |

Löslichkeit: schlecht löslich in unpolaren Lösungsmitteln, sehr gut löslich in polaren Lösungsmitteln;
nachweisbare Bestandteile:

| Substanzen | | Verhältnis (Näherung) |
|---|---|---|
| Neutralzucker (Glucose, Galactose, Ribose) | (≤) | 1 |
| Aminozucker (Glucosamin, Muraminsäure) (nach Hydrolyse) | | 3,5 |
| Aminosäuren(Ala, Glu,iso-Gln, Gly, Lys, DAP) | | 4 |
| Lipide | | <1 |
| Phosphor | | <1 |

Strucktur:
Peptidoglycanstruktur, deren Glycangerüst aus 10 bis 80 Disacchariden aus N-Acetylglucosaminyl-N-acetylmuramyl besteht.

**Beispiel 2**

Herstellung des Hilfsmittels "Cocktail"

1000 ml Ethanol 96 % und
40 g Acetaldehyd puriss. werden gemischt.
Eine Wirkdosis sind 25 ml davon.
Applikationsanleitung:
25 ml des Cocktails werden mit 225 ml Wasser gemischt und oral verabreicht.
Die Tagesdosis beträgt 2 bis 3 Wirkdosen, auf ärztliche Anordnung kann höher dosiert werden.
Der Cocktail darf bei Verdacht auf Hirnmetastasen nicht verabreicht werden.

Herstellungsbeispiele für Jomol-Derivate

**Beispiel 3**

Jomol-Sn (wird auch als Jomo-tech bezeichnet):

(a) Allgemeine Arbeitsbedingungen:
Unter Stickstoff bei 0 bis 10 °C arbeiten, Lösungen vor Verwendung mit Stickstoff durchblasen, Reaktionsgefäße säuregewaschen verwenden.
(b) 20 mg Jomol werden in 100 µl 1 N HCl gelöst und dann wird die Lösung 10 bis 15 Minuten stehengelassen. Danach wird 1 mg $SnCl_2 \times 2H_2O$ (fest) zugegeben und das Reaktionsgemisch ca. 20 Minuten stehengelassen. Es werden 2 ml einer 0,1 N HCl, physiologischen Kochsalzlösung mit 50 µg $FeCl_2 \times 4H_2O$ (0 bis 1 °C) zugegeben.

Nach kräftigem Umschütteln werden je 20 µl der Lösung bei einer Stellflächentemperatur von -10 bis -20 °C in vorgekühlte Durchstechflaschen eingefüllt. Die Durchstechflaschen werden sofort zugedekkelt und bis zum Gebrauch gefroren (ca. -20 °C) aufbewahrt.

**Beispiel 4**

Mit $^{99m}$Technetium beladenes Jomol-Sn für die Diagnostik

Zur Markierung mit $^{99m}$Technetium wird in die gefrorene Durchstechflasche (Beispiel 3) 20 bis 100 mCi $^{99m}$Tc in einem Volumen bis zu 5 ml zugegeben (aus einem $^{99m}$Molybdängenerator, Squibb von Heyden,

Regensburg, FRG). Vorzugsweise wird das zweite Eluat des Präparationstages verwendet. Nach 10 bis 15 Minuten bei Raumtemperatur (volles Licht vermeiden) ist das Präparat zur i.v.-Injektion für einen diagnostischen Test mit der Gammakamera verwendbar.

**Beispiel 5**

Jomol-Sn lyo (wird auch als Jomo-tech lyo bezeichnet), geeignet zur Beladung mit $^{99m}$Technetium für die Diagnostik

(a) Allgemeine Arbeitsbedingungen:
Unter Stickstoff arbeiten, Reaktionsgefäße säuregewaschen verwenden.
(b) 6 mg Jomol, aus 10 mM Tris/HCl-Puffer, pH 7 bis 7,5, lyophilisiert, werden in 100 µl 1 N HCl gelöst und ca. 15 Minuten bei Raumtemperatur stehengelassen. Danach werden 0,3 mg $SnCl_2 \times 2H_2O$ in 100 µl 1 N HCl gelöst und zu der Jomollösung gegeben. Dann wird ca. 20 Minuten bei Raumtemperatur stehengelassen.
(c) 0,6 mg Natriumacetat, 3,4 mg Bernsteinsäure und 50,0 mg Laktose werden in 60 ml physiologischer Kochsalzlösung bei 0 ° C gelöst. 0,8 ml dieser Lösung werden zu der gemäß (b) erhaltenen Jomollösung gegeben. Es entsteht ein Endvolumen von 1 ml.

Die Einzeldosierung je 100 µl erfolgt in 10 vorgekühlte Durchstechflaschen auf einer -10 bis -20 ° C kalten Stellfläche. Danach wird lyophilisiert, unter Stickstoff verschlossen und bei -20 ° C bis zum Gebrauch aufbewahrt.

Anmerkung: Die oben genannten Lösungen werden stickstoffdurchblasen eingesetzt.

**Beispiel 6**

Mit $^{99m}$Technetium beladenes Jomol-Sn lyo

Die Markierung mit $^{99m}$Technetium erfolgt auf gleiche Weise wie in Beispiel 4 beschrieben.

**Beispiel 7**

Kopplungsprodukt aus Jomol und Diethylentriaminpentaessigsäure

Dieses Kopplungsprodukt ist zur Beladung mit $^{111}$Indium oder mit durch $SnCl_2$ reduziertem $^{99m}$Technetium geeignet.
(a) Allgemeine Arbeitsbedingungen:
Die Reaktionsgefäße werden mit Säure gewaschen verwendet, es wird bei 0 bis 10 ° C unter Stickstoff gearbeitet, die verwendeten Lösungen sind stickstoffdurchblasen.
(b) 5 mg Diethylentriaminpentaessigsäure-Anhydrid werden in 5 ml absolutem Diethylether suspendiert (diese Suspension wird als S1 bezeichnet).
(c) Ca. 200 µg Jomol werden in 300 µl Bicarbonatpuffer (0,05 M, pH 7,0) in physiologischer Kochsalzlösung gelöst. Unter starkem Rütteln (Mixer, 30 bis 60 Sekunden) werden 25 µl der Suspension S1 zugegeben und dann wird 1 Stunde bei 37 ° C inkubiert. Der Ether dunstet hierbei ab.

Es folgt eine Chromatographie:
FPLC-Chromatographie:
Säule: Superose 12, HR 10/30, Code-Nr. 17-0538-01, Pharmacia, Freiburg, FRG.
Aufgetragenes Volumen: ca. 300 µl
Laufmittel: physiologische Kochsalzlösung
Flußrate: 1 ml/min
Detektion: $\lambda$ = 214 nm, Uvicon 820, Kontron,
HPLC-Pumpe: LC Pumpe 410, Kontron, München, FRG.

In der Produktfraktion liegt Jomol vollständig als Jomol-DTPA vor. Die Bindung des DTPA an der Aminogruppe des Lysin des Jomols ist stabil, andere Anbindungen hydrolysieren.

**Beispiel 8**

Markierung von Jomol-Diethylendiaminpentaessigsäure mit $^{111}$Indium

Zu ca. 220 µg Jomol-Diethylentriaminpentaessigsäure (im folgenden als Jomol-DTPA bezeichnet) in 3 ml 5 mM Bicarbonatpuffer (pH 7,0) in physiologischer Kochsalzlösung werden ca. 2 mCi $^{111}$Indium in 0,2 ml 40 mM HCl zugegeben. Nach Umschütteln und 15 Minuten Abwarten be Raumtemperatur ist die erhaltene Lösung für eine intravenöse Injektion (i.v.) verwendbar. Die Prüfung erfolgt mit der Gammakamera.

Das mit $^{111}$In markierte Jomol-DTPA führt bei FPLC-Chromatographie (214 nm, 0,1 M Phosphatpuffer) zu dem in der Figur 4 gezeigten Elutionsmuster. Im Diagramm ist die Verteilung der Radioaktivität auf drei separierbare Fraktionen angegeben.

**Beispiel 9**

Markierung von Jomol-Diethylendiaminpentaessigsäure mit $^{99m}$Technetium

Zur Markierung mit $^{99m}$Technetium wird zu 1 ml Jomol-DTPA-Lösung (ca. 50 µg) (gefroren) 100 µl einer salzsauren (0,1 N HCl) 3 mM $SnCl_2 x 2H_2O$ physiologischen Kochsalzlösung stabilisiert durch 0,1 mM $FeCl_2 x 4H_2O$ gegeben und 10 Minuten abgewartet. Danach werden 20 bis 50 mCi $^{99m}$Technetium in einem Volumen bis zu 5 ml zugegeben (vom zweiten Eluat des Präparationstages). Nach 10 bis 15 Minuten ist das Präparat zur i.v.-Injektion zum Test an der Gammakamera verwendbar.

**Beispiel 10**

Mit radioaktivem Jod markiertes Jomol

Die Jodierung des Jomols erfolgt nach der modifizierten Chloramin-T-Methode bei 0 bis 5 °C.

250 µg Jomol, lyophilisiert aus 10 mM $NH_4$-Acetat, werden in 60 µl PBS (0,25 M, pH 7,5) gelöst. Dann wird 1 mCi $^{131}$J (Amersham-Buchler, Braunschweig, FRG) in 25 µl zugegeben, danach 53 µg Chloramin T (N-Chloro-p-toluene-sulfonamid-$Nax3H_2O$, Serva, Heidelberg, FRG, 16784) in 15 µl PBS (0,25 M). Nach ca. 30 Sekunden wird 90 µg Na-Metabisulfit in 20 µl PBS (0,25 M) zugegeben.

Es folgt eine Chromatographie über Sephadex G 25 (Säulenbett 6 cm × 0,6 cm, Laufmittel 0,1 N Essigsäure, Säule mit ca. 50 µg Jomol aequilibiert). Die Ausbeute liegt bei 80 %.

**Beispiel 11**

Herstellung von Jomol-Kopplungsprodukten

(a) Jomol-Uridin (Jomo-J/U)
Die Herstellung erfolgt analog der Anbindung von Diethylentriaminpentaessigsäure, wie in Beispiel 7 beschrieben.

Es werden 30 µg Uridin-2',3'-dialdehyd in 10 µl getrocknetem Diethylenether gelöst (Uridin-2',3'-dialdehyde, Sigma, U 0128). Das erhaltene Produkt wird, wie in Beispiel 7 beschrieben, chromatographiert.

(b) Jomol-L-Tyrosin (Jomo-J/TS)
Die Herstellung des Kopplungsproduktes erfolgt auf gleiche Weise, wie in Beispiel 7 beschrieben, wobei α-Amino-β-[p-hydroxyphenyl]-propionaldehyd in einer Menge von 30 µg in 10 µl absolutem Diethylether verwendet werden.

(c) Jomol-L-Thyronin (Jomol J/Tn)
Die Anbindung von α-Amino-β-[p-hydroxyphenyl(p-hydroxyphenylether)]-propionaldehyd erfolgt auf gleiche Weise wie in Beispiel 7 beschrieben.

**Beispiel 12**

Herstellung eines mit Jod markierten Kcpplungsproduktes aus Jomol-Uridin/Jomol-L-Tyrosin/Jomol-L-Thyronin/ Jomo-Color

Das gemäß Beispiel 11 (a) hergestellte Kopplungsprodukt aus Jomol und Uridin wird mit einem geeigneten Radionuclid nach dem für Uridin üblichen Verfahren von entsprechenden Laboratorien (z.B. Amersham, GB) durchgeführt.

Die gemäß den Beispielen 11 (b) und 11 (c) hergestellten Kopplungsprodukte und Jomo-Color (vgl. Beispiel 15) werden nach dem in Beispiel 10 angegebenen Verfahren mit radioaktivem Jod markiert.

**Beispiel 13**

Jomol gebunden an eine Vorstufe des Kryptofix (Jomo-Ra bzw. Jomol-CE)

Zu 200 µl einer wäßrigen Lösung von 1 mg Jomol gibt man 1 ml 0,2 M Boratpuffer (pH 8,0). Getrennt wird eine Lösung von einer Vorstufe des Kryptofix hergestellt. Die Vorstufe wird als CE bezeichnet und ist von der Firma Merck in Darmstadt erhältlich. Das CE besitzt die folgende Formel

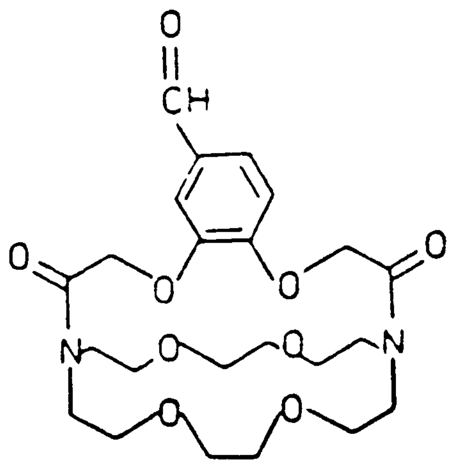

Die Lösung besitzt eine Konzentration von 1 mg pro ml Ethanol.

240 µl der ethanolischen CE-Lösung werden zu der obigen Jomollösung zugegeben. Zu dem Reaktionsgemisch gibt man 160 µl Cyanoborhydridlösung (1 mg/ml 0,2 M Boratpuffer, pH 8,0). Das Reaktionsgemisch wird 20 Stunden bei 37 ° C inkubiert und kann dann direkt zur Anbindung von radioaktivem Marker verwendet werden.

**Beispiel 14**

Markierung von Jomol-CE mit radioaktivem Radium

Nach der obengenannten Inkubation wird zum Reaktionsgemisch $^{224}$Radium als $^{224}RaCl_2$-Lösung (Amersham/Buchler, Braunschweig, FRG) bis 200 µCi gegeben und bei Raumtemperatur ca. 1 Stunde abgewartet. Die erhaltene Lösung wird auf eine Superose$^{T}$H-FPLC-Säule (Pharmacia, Freiburg, FRG) gegeben. Als Laufmittel wird 50 mM Kaliumphosphatpuffer von pH 6,8 in physiologischer Kochsalzlösung, 1 ml/min verwendet. Detektion bei 214 nm, und Radioaktivitätsmonitoring (Durchlaufmonitor, Berthold, Wildbad,FRG).

Das Reaktionsprodukt und freies Jomol (0,5 mg/ml Wasser) weisen im wesentlichen das gleiche Spektrum auf. Die fraktionierte Entnahme erlaubt die Abtrennung der niedermolekularen Komponenten des Reaktionsgemisches. Das Produkt wird in 2 Peaks eluiert. Nach Austritt von ca. 80 % der aufgetragenen Radioaktivität wird am 2. Peak eine Schulter erkennbar. Die dieser Schulter zuzuordnenden Fraktionen und die nachfolgenden werden verworfen. Diese enthalten unter anderem das Cyanoborhydrid.

Da Jomol-CE-$^{224}$Ra nicht nur an Krebszellenoberflächen angereichert wird (wo bei Gabe von ca. 5 µCi i.v. ca. 1 g Krebsgewebe zerstört wird), sondern auch, wie das immunstimulierende reine Immunmodulatormolekül, in Monozyten, Makrophagen, Mikrophagen und Knochenmarksstammzellen geht, muß vor der Verabreichung von Jomol-CE-$^{224}$Ra i.v. darauf geachtet werden, daß diese Zellen das Produkt nicht aufnehmen können. Eine Beschränkung der Aufnahme von Jomol-CE-$^{224}$Ra in immunkompetente und Knochenmarksstammzellen ist mit hohen Dosen Cortisol i.v./i.m. in dem Fachmann geläufigem Abstand vor der Gabe der Aktivität erforderlich, weitere Maßnahmen sind durch Hinzuziehen eines Immunologen ad hoc zu ergreifen. Bei Unterlassung droht eine Leukämie. Das Medikament sollte hauptsächlich nach Mikroaussaat von Tumorzellen durch Operation eingesetzt werden (nicht bei Non-Hodgkin-Tumoren).

**Beispiel 15**

Jomol-Fluoresceinisothiocyanat (Jomo-Color) zur Diagnostik und/oder Therapie

Arbeitsbedingungen: Raumtemperatur
Fluoresceinisothiocyanat in gesättigter Lösung in Ethanol 96%
Jomol 100 mg in 200 µl $H_2O$ gelöst.

Jomol und Fluoresceinisothiocyanat werden im molaren Verhältnis 1:5 zusammengegeben und 30 Sekunden im Ultraschall gemischt. Dann ca. 10 Minuten abwarten und über eine Säule 30 cm × 0,6 cm, Säulenbett 15 cm Sephadex G 75, Laufmittel physiologische Kochsalzlösung 0,5 ml/min, fraktionieren. Fraktion 2,5 bis 9 ml enthält Jomo-Color.
Die Säule ist vorher mit 2 Läufen mit je 5 mg Jomol-Fitc zu aequilibrieren.

Die erhaltene Fraktion wird entweder sofort bei -20 °C eingefroren, oder je 300 µg Jomol-Fitc portioniert und lyophilisiert, unter Stickstoff verschlossen und bis zum Gebrauch bei -20 °C aufbewahrt.

Beispiele für Kits und ihre Anwendungen

(1) Jomol-Kit zur Immunstimulation

Bestandteile:

(a) Cocktail (Ethanol 96%, 50 g/Acetaldehyd puriss., 2 g)
2 × 1 Dosis, Schraubdeckelflasche
(b) Jomol (300 - 600 µg Lyophilisat ≙ 30 µg Trockensubstanz)
1 Dosis, Durchstechflasche
(c) Physiologische Kochsalzlösung (5 ml) Lösungsmittel für Jomol
Ampulle

Anwendungsanweisung:

zu (a) Cocktail: (nicht bei Hirnmetastasen)
1 Dosis = 25ml aus der Schraubdeckelflasche Cocktail mit 225 ml Wasser verdünnen und oral verabreichen. 30 Minuten warten, dann
zu (b) Jomol: Die physiologische Kochsalzlösung wird in einer Spritze aus der Ampulle (c) aufgenommen und in die Durchstechflasche (b) gegeben. Nach Auflösen des Lyophilisats Jomol unter Schwenken der Durchstechflasche wird die Lösung wieder in der Spritze aufgenommen und dem Patienten intravenös injiziert.

(2) Jomol-Aer-Kit zur Immunstimulation per inhalationem

Bestandteile:

(a) Cocktail (siehe (1a))
(b) Jomol (siehe (1b))
(c) physiologische Kochsalzlösung, 5 ml, Durchstechflasche, enthaltend Liposomen, 5 mg, bestehend aus Phosphatidylcholin : Phosphatidylserin : Cholesterol im molaren Verhältnis 8:2:10

Anwendungsanweisung:

zu (a) Cocktail (siehe (1a))
zu (b) u. (c) Die Durchstechflaschen (b) und (c) werden mit einer Doppelkanüle verbunden, der Inhalt von (c) läuft in (b). Die Durchstechflasche (b) wird geschüttelt, der Inhalt wird in ein Inhalatorgefäß gegeben und zum Beispiel mit einem Siemens-Mikroinhalator inhaliert.

(3) Jomo-tech, Kit zur szintigraphischen Darstellung von malignen Tumoren mit der Gammakamera

Bestandteile:

(a) Cocktail (siehe (1a)
(b) Jomo-tech (200 µg Jomol-Sn ≙ 20 µg Trockensubstanz, enthaltend Jomol + 10 µg $SnCl_2 \times 2\,H_2O$ +

0,5 µg $FeCl_2 \times 4\ H_2O$ in 0,1 N HCl physiologischer Kochsalzlösung), Durchstechflasche, gefroren bei -20 °C

Anwendungsanweisung:

zu (a) Cocktail (siehe (1a)
zu (b) Jomotech: 20 bis 30 mCi $^{99m}$Technetium in physiologischer Kochsalzlösung (zweites Eluat des Präparationstages aus einem $^{99m}$Molybdängenerator), in einem Volumen bis zu 5 ml, werden in die gefrorere Durchstechflasche (b) gegeben mit einer Spritze, die man darauf stecken läßt. Die Durchstechflasche (b) wird mehrmals umgeschüttelt, 10 bis 15 Minuten bei Raumtemperatur stehen gelassen, dann wird intravenös injiziert.

(4) Jomo-tech lyo, Kit zur szintigraphischen Darstellung von malignen Tumoren mit der Gammakamera

Bestandteile:

(a) Cocktail (siehe (1a))
(b) Jomo-tech lyo (1350 µg Jomol-Sn ≙ 813µg Trockensubstanz, enthaltend Jomol + 30 µg $SnCl_2 \times 2\ H_2O$ + 8 µg Na-Acetat + 45 µg Bernsteinsäure + 670 µg Lactose), lyophilisiert, Durchstechflasche, gefroren bei -20 °C

Anwendungsanweisung:

siehe (3) Jomo-tech

(5) SmiL-N/Tc, Kit zur szintigraphischen Darstellung von malignen Tumoren der Lunge mit der Gammakamera

Bestandteile:

(a) Cocktail (siehe (1a))
(b) Jomo-tech (siehe (3b))
(c) Liposomen in physiologischer Kochsalzlösung (siehe (2c))

Anwendungsanweisung:

zu (a) Cocktail (siehe (1a))
zu (b) Jomo-tech (siehe (3b)). Anstelle der intravenösen Injektion wird in die Durchstechflasche (c) instilliert.
zu (c) Inhalt von (b) und (c) wird zusammengebracht, umgeschüttelt und mit einem geschlossenen Inhalationssystem inhaliert.

(6) Jomo-In, Kit zur szintigraphischen Darstellung von malignen Tumoren mit der Gammakamera

Bestandteile:

(a) Cocktail (siehe (1a))
(b) Jomo-In (200 µg Jomol, trocken, + 20 µg DTPA = 220 µg Jomol-DTPA in 3 ml physiologischer Kochsalzlösung, 5 mM Na-Bicarbonatpuffer, pH 7,0), Durchstechflasche, gefroren bei -20 °C

Anwendungsanweisung:

zu (a) Cocktail (siehe (1a))
zu (b) Jomo-In auftauen und 2 bis 3 mCi $^{111}$Indium in 0,2 bis 0,3 ml 40 mM HCl zugeben ($InCl_3$) Nach Umschütteln und 15 Minuten Abwarten bei Raumtemperatur ist die erhaltene Lösung für eine intravenöse Injektion zum Test an der Gammakamera verwendbar.

(7) Jomo-J, markiert mit $^{131}$Jod, Kit zur szintigraphischen Darstellunq von malignen Tumoren mit der Gammakamera und zur Therapie

Bestandteile:

(a) Cocktail (siehe (1a))
(b) Jomo-J, markiert mit $^{131}$Jod (ca. 250 µg Jomol ≙ 25 µg Jomol-Trockensubstanz + 1 mCi $^{131}$Jod) in 1 bis 2 ml PBS, 25 mM, 0,05 N Essigsäure, Durchstechflasche, gefroren bei -20 °C

Anwendungsanweisung:

zu (a) Cocktail (siehe (1a))
zu (b) Jomo-J, markiert mit $^{131}$Jod, auftauen und intravenös injizieren, danach folgt der Test an der Gammakamera
Eine Blockierung der Schilddrüse mit Natriumperchlorat wird empfohlen.

Zur Therapie werden auf Anordnung des behandelnden Arztes gegebenenfalls höhere Dosen eingesetzt.

(8) Jomo-Ra,markiert mit $^{224}$Radium, Kit zur lokalen Strahlentherapie von Spurenaussaaten von Krebszellen (JomolCE-$^{224}$Ra für i.v./i.t.)

Bestandteile:

(a) Cocktail (siehe (1a))
(b) Jomol-CE-$^{224}$Ra (ca 30 µg Jomol-E-Trockensubstanz, markiert mit 200 µCi $^{224}$Radium) in 2 ml PBS, 25 mM, Durchstechflasche, gefroren bei -20 °C

Anwendungsanweisung:

zu (a) Cocktail (siehe (1a))
zu (b) Jomol-CE-$^{224}$Ra:
Zwei Stunden vor der Behandlung mit Jomo-Ra 250 mg Cortisol i.v., dann Cocktail oral, dann (b) auftauen und intravenös oder intratumoral applizieren.

(9) Jomo-Color, Kit zur intraoperativen Farbdiagnostik mit ultraviolettem Licht und zur Therapie bei malignen Tumoren

Bestandteile:

(a) Cocktail (siehe (1a))
(b) Jomol-Fluoresceinisothiocyanat (ca. 300 µg Trockensubstanz, enthält Jomol:Fluoresceinisothiocyanat im molaren Verhältnis 1:5) aus 5 ml physiologischer Kochsalzlösung, lyophilisiert, Durchstechflasche, gefroren bei -20 °C

Anwendungsanweisung:

zu (a) Cocktail bei Anwendung zur Thera-pie (siehe (1a)); bei Anwendung zur intraoperativen Diagnostik werden 5 bis 10 ml des Cocktails zur Herstellung einer Infusionslösung, die 10 bis 30 Minuten vor der i.v.-Gabe von (b) verabreicht wird, benutzt.

Es ist zu beachten, daß Morphin und seine Derivate durch den Cocktail in ihrer Wirkung 30- bis 40fach gesteigert werden.

zu (b) Jomol-Fluoresceinisothiocyanat wird mit 5 ml $H_2O$ (Ampuwa ®) gelöst und intravenös verabreicht.

Zur intraoperativen Diagnostik 10 bis 20 Minuten p.a. von (b) ist eine UV-Leuchte erforderlich (siehe Augenheilkunde, Zahnheilkunde).

(10) Jomo-Lab zur Schnelldiagnostik maligner Tumoren in vitro

Bestandteile:

Lyophilisat in Laborreinheitsgrad, nicht zur Anwendung in vivo. 300 µg Fluoresceinisothiocyanat-Jomol, 45 mg NaCl, Schraubdeckelflasche, gefroren bei -20 °C

Anwendungsanweisung:

In die Schraubdeckelflasche werden 5 ml $H_2O$ gegeben, die Gewebsprobe wird dazugegeben, 5 Minuten bei Raumtemperatur abgewartet, die Gewebsprobe in physiologischer Kochsalzlösun gewaschen.

Im Anschnitt der Gewebsprobe zeigt sich im ultravioletten Licht mit Lupe oder im Fluoreszenzmikroskop das Tumorgewebe hell, das gesunde Gewebe dunkel. Der op.-Präparationsrand zeigt durch zellzerstörungsbedingte Proteasenfreisetzung ebenfalls Fluoreszenz.

**Patentansprüche**

1. Jomol, charakterisiert durch folgende Eigenschaften:
   (1) Absorptionsmaximum im UV-Spektrum bei 282,5 nm;
   (2) apparente Molekulargewichte bei 4500, 3000 und 900;
   (3) starke Hygroskopizität;
   (4) je nach Restwassergehalt elfenbeinfarbig amorphkristallin-flockig;
   (5) pH-Wert von 5,5 (1 mg der Verbindung, gelöst in 1 ml Wasser);
   (6) schwach positive, rosa Ninhydrin-Reaktion;
   (7) anfärbbar mit Orcin Reagens;
   (8) Peptidanteil: ca. 50%, bezogen auf die amorphe Substanz (Proteintest MicroBioRad nach L. Thomas, J. Clin. Chem. Clin. Biochem., Bd. 19, 1981, Seite 203-208, Fa. BioRad, München; Coomassie Brilliant Blue G 250®;
   (9) folgende $R_f$-Werte (Lösungsmittelangabe Volumen:Volumen)

| | | |
|---|---|---|
| $R_f$ = 0,20 | (Butanol:Eisessig:Wasser | 4:1:1) |
| $R_f$ = 0,45 | (Benzol:Eisessig:Wasser | 2:1:2) |
| $R_f$ = 0,685 | (Methanol:Eisessig:Wasser | 4:1:1) |
| keine Wanderung | (Chloroform:Methanol | 96:4) |
| $R_f$ = 0,54 | (Chloroform:Methanol | 1:1); |

   (10) Löslichkeit: schlecht löslich in unpolaren Lösungsmitteln, sehr gut löslich in polaren Lösungsmitteln;
   (11) nachweisbare Bestandteile:

| Substanzen | Verhältnis (Näherung) |
|---|---|
| Neutralzucker (Glucose, Galactose, Ribose) | (≤) 1 |
| Aminozucker (Glucosamin, Muraminsäure) (nach Hydrolyse) | 3,5 |
| Aminosäuren (Ala, Glu, iso-Gln, Gly, Lys, DAP) | 4 |
| Lipide | <1 |
| Phosphor | <1 |

   (12) Struktur:
   Peptidoglycanstruktur, deren Glycangerüst aus 10-80 Disacchariden aus N-Acetylglucosaminyl-N-acetylmuramyl besteht.

**2.** Verfahren zur Herstellung von Jomol nach Anspruch 1 durch Züchten eines Stammes von Nocardia-opaca-Zellen und Gewinnen der Zellen aus der Kultur, dadurch **gekennzeichnet**, daß man
(a) Norcardia-opaca-Zellen (DSM 43 202 bzw. ATCC 21953) auf einem gepufferten Nährmedium züchtet,
(b) die Zellen gewinnt,
(c) die Zellen in einem Glucose und Puffer enthaltenden Medium suspendiert, die Suspension 15 Minuten bis einige Stunden stehen läßt,
(d) die Suspension in einem Puffer mit einem murolytischen Enzym, vorzugsweise Lysozym, und gegebenenfalls mit Desoxyribonuclease behandelt,
(e) die Zellen zerstört,
(f) das erhaltene Material in Bodensatz und Überstand trennt,
(g) den Überstand an Sephadex-Säulen unter Verwendung eines Puffers als Eluierungsmittel, der gegebenenfalls 0,1 bis 0,05% EDTA-$Na_2$ enthalten kann, der Trennung unterwirft,
(h) die aktive Fraktion gewinnt und
(i) die aktive Fraktion mit Acetaldehyd umsetzt,
wobei als Puffer Kaliumphosphatpuffer, Tris-HCl-Puffer, Ammoniumacetatpuffer und andere verwendet werden können.

**3.** Verfahren nach Anspruch 2, dadurch **gekennzeichnet**, daß man vorzugsweise auf TraubenzuckerAgar-(2%)-Platten die Bakterien 5 bis 8 Tage anzüchtet und bei der Stufe (a) als Nährmedium Nährbouillon verwendet und die Züchtung während einer Zeit von 10 bis 20 Stunden bei einer Temperatur von 30 bis 37 °C durchführt.

**4.** Verfahren nach Anspruch 2 oder 3, dadurch **gekennzeichnet**, daß nach der Stufe (b) und vor der Bebrütung mit Glucose ein- oder mehrmals mit Tris-HCl-Puffer, pH 7,4, der gegebenenfalls 0,1 bis 0,05% EDTA-$Na_2$ enthalten kann, gewaschen wird.

**5.** Verfahren nach einem der Ansprüche 2, 3 oder 4, dadurch **gekennzeichnet**, daß man als aktive Fraktionen solche gewinnt, die im UV-Spektrum eine Absorption bei 214 nm zeigen.

**6.** Verfahren nach einem der Ansprüche 2, 3, 4 oder 5, dadurch **gekennzeichnet**, daß die Umsetzung der aktiven Fraktion mit Acetaldehyd in einem Molverhältnis von 1 Mol aktiver Fraktion (angenommenes mittleres Molekulargewicht: 4000) mit 1,8 bis 2,2 Mol Acetaldehyd erfolgt.

**7.** Jomol, dadurch **gekennzeichnet**, daß es nach einem der Ansprüche 2 bis 6 erhalten worden ist.

**8.** Pharmazeutisch annehmbares Jomolderivat, dadurch **gekennzeichnet**, daß es Jomol nach Anspruch 1 und daran gekoppelt einen Kronenether, Diethylentriaminpentaessigsäure, Uridin, L-Thyronin, L-Tyrosin, Fluoresceinisothiocyanat oder Zinn(II)-chlorid enthält.

**9.** Markiertes Jomol oder markiertes Jomolderivat, dadurch **gekennzeichnet**, daß es Jomol nach Anspruch 1 oder eines seiner pharmazeutisch annehmbaren Derivate nach Anspruch 8, markiert mit $^{99m}$Technetium, $^{111}$Indium, $^{123}$Jod, $^{125}$Jod, $^{130}$Jod, $^{131}$Jod, $^{132}$Jod oder $^{224}$Radium, einem Farbstoff oder einem Cytostatikum, enthält.

**10.** Jomol nach Anspruch 1, pharmazeutisch annehmbares Jomolderivat nach Anspruch 8, markiertes Jomol oder markiertes Jomolderivat nach Anspruch 9, dadurch **gekennzeichnet**, daß es
(i) in und/oder auf Liposomen
oder
(ii) lipidiert
vorliegt.

**11.** Verfahren zur Herstellung eines pharmazeutisch annehmbaren Jomolderivats nach Anspruch 8, dadurch **gekennzeichnet**, daß man eine Jomollösung in einem polaren Lösungsmittel mit einer wasserfreien Lösung oder Suspension, einer reaktiven Vorstufe eines Kronenethers, Diethylentriaminpentaessigsäureanhydrid, einer reaktiven Vorstufe von Uridin, L-Thyronin, L-Tyrosin oder Fluoresceinisothiocyanat oder einer salzsauren wäßrigen Lösung von Zinn(II)-chlorid bei einer Temperatur im Bereich von 15 bis 40 °C während einer Zeit von 5 min bis 60 min umsetzt und das Produkt in

an sich bekannter Weise durch Chromatographie reinigt und isoliert.

**12.** Verfahren zur Herstellung von markiertem Jomol oder einem markierten Jomolderivat nach Anspruch 9, dadurch **gekennzeichnet**, daß man eine Jomollösung in einem polaren Lösungsmittel mit einer Lösung eines radioaktiven Alkalijodids in Anwesenheit von Chloramin T umsetzt und gegebenenfalls die erhaltene Lösung reinigt oder daß man eine Jomollösung in einem polaren Lösungsmittel mit einer Lösung oder Suspension einer reaktiven Vorstufe eines Farbstoffs oder Cytostatikums in einem wasserfreien Lösungsmittel bei einer Temperatur im Bereich von 15 bis 40 ° C während einer Zeit von 5 min bis 60 min umsetzt oder daß man eine wäßrige Lösung eines Jomolderivats nach Anspruch 8 mit $^{99m}$Technetium, $^{111}$Indium, $^{123}$Jod, $^{125}$Jod, $^{130}$Jod, $^{131}$Jod, $^{132}$Jod oder $^{224}$Radium in an sich bekannter Weise markiert.

**13.** Diagnostisches Mittel, dadurch **gekennzeichnet**, daß es
Jomol nach Anspruch 1 oder 7 oder
Jomol oder ein Jomolderivat nach Anspruch 8, markiert mit einem Radioisotop und/oder einem Farbstoff,
sowie gegebenenfalls übliche Trägerstoffe und/oder Verdünnungsmittel enthält.

**14.** Diagnostisches Mittel nach Anspruch 13, dadurch **gekennzeichnet**, daß das Jomol oder das Jomolderivat mit $^{99m}$Technetium, $^{111}$Indium, $^{123}$Jod, $^{125}$Jod, $^{130}$Jod, $^{131}$Jod, $^{132}$Jod oder $^{224}$Radium als Radioisotop markiert ist.

**15.** Arzneimittel, dadurch **gekennzeichnet**, daß es Jomol nach Anspruch 1 oder markiertes Jomol oder ein markiertes Jomolderivat nach Anspruch 9, markiert mit einem Farbstoff, einem Cytostatikum oder einem Radioisotop, sowie gegebenenfalls übliche Trägerstoffe und/oder Verdünnungsmittel enthält.

**16.** Arzneimittel nach Anspruch 15, dadurch **gekennzeichnet**, daß das Jomol oder das Jomolderivat mit $^{99m}$Technetium, $^{111}$Indium, $^{123}$Jod, $^{125}$Jod, $^{130}$Jod, $^{131}$Jod, $^{132}$Jod oder $^{224}$Radium als Radioisotop markiert ist.

**17.** Erzeugnis, enthaltend
(1) ein diagnostisches Mittel nach Anspruch 13 oder 14 oder ein Arzneimittel nach Anspruch 15 oder 16 und
(2) ein Hilfsmittel, welches einen Aldehyd der Formel I

$$RCHO \quad (I)$$

worin R ein Wasserstoffatom und eine geradkettige oder verzweigte Kohlenwasserstoffgruppe mit 1 bis 4 Kohlenstoffatomen bedeutet, wobei der freie Aldehyd auch direkt oder indirekt von Stoffen metabolisch freigesetzt werden kann, und
gegebenenfalls einen Alkohol der Formel II

$$R^1CH_2OH \quad (II)$$

worin $R^1$ die oben für R angegebene Bedeutung besitzt, sowie gegebenenfalls übliche pharmazeutisch annehmbare Trägerstoffe und/oder Verdünnungsmittel enthält,
zur gleichzeitigen, getrennten oder zeitlich abgestuften Verwendung bei der Diagnose und Therapie von malignen Tumoren und zur Therapie von Immunabwehrschwächen.

**18.** Erzeugnis nach Anspruch 17, dadurch **gekennzeichnet**, daß es zwei Behälter umfaßt, wobei im ersten Behälter die Komponente (1) und im zweiten Behälter die Komponente (2) vorhanden sind.

**19.** Erzeugnis nach Anspruch 17, dadurch **gekennzeichnet**, daß R und $R^1$ in den Formeln I und II eine Methylgruppe bedeuten.

**20.** Erzeugnis nach Anspruch 17, 18 oder 19, dadurch **gekennzeichnet**, daß das Hilfsmittel den Aldehyd und den Alkohol im Verhältnis von 15 bis 40 g Aldehyd auf 1000 g Alkohol enthält.

**21.** Erzeugnis nach einem der Ansprüche 17 bis 20, dadurch **gekennzeichnet**, daß die Verbindung (1) in einer für die orale oder parenterale Verabreichung geeigneten Form und das Hilfsmittel (2) in einer für die orale oder parenterale Verabreichung geeigneten Form vorliegen.

**22.** Erzeugnis nach einem der Ansprüche 17 bis 20, dadurch **gekennzeichnet**, daß die Verbindung (1) in einer für die Verabreichung durch Inhalation geeigneten Form und das Hilfsmittel (2) in einer für die orale Verabreichung geeigneten Form vorliegen.

**23.** Erzeugnis nach einem der Ansprüche 17 bis 22, dadurch **gekennzeichnet**, daß es einen dritten Behälter mit einer Komponente umfaßt, welche einen pharmazeutisch annehmbaren Träger enthält.

**24.** Erzeugnis nach Anspruch 23, dadurch **gekennzeichnet**, daß die Komponente (2) zusätzlich ein Peroxid, wie Wasserstoffperoxid, enthält.

## Claims

**1.** Jomol, characterized by the following properties:
(1) Absorption maximum in UV spectrum at 282.5 nm;
(2) apparent molecular weights at 4500, 3000 and 900;
(3) strong hygroscopicity;
(4) depending on the residual water content, ivory coloured, amorphous-crystalline-flocculent;
(5) pH 5.5 (1 mg of compound dissolved in 1 ml of water);
(6) slightly positive, pink ninhydrin reaction;
(7) can be coloured with orcin reagent;
(8) peptide content: about 50%, based on the amorphous substance
(Protein test MicroBioRad according to L. Thomas, J. Clin. Chem. Clin. Biochem., Volume 19, 1981, pages 203-208, BioRad Company, Munich; Coomassie Brilliant Blue G 250(R);
(9) the following $R_f$-values (solvent given in volume:volume)

| | |
|---|---|
| $R_f$ = 0.20 | (butanol:glacial acetic acid:water 4:1:1) |
| $R_f$ = 0.45 | (benzene:glacial acetic acid:water 2:1:2) |
| $R_f$ = 0.685 | (methanol:glacial acetic acid:water 4:1:1) |
| no migration | (chloroform:methanol 96:4) |
| $R_f$ = 0.54 | (chloroform:methanol 1:1); |

(10) Solubility: sparingly soluble in apolar solvents, very readily soluble in polar solvents;
(11) Detectable components:

| Substances | Ratio (approximate) |
|---|---|
| Neutral sugars (glucose, galactose, ribose) | (≦) 1 |
| Amino sugars (glucosamine, muramic acid) (after hydrolysis) | 3.5 |
| Amino acids (Ala, Glu, iso-Gln, Gly, Lys, DAP) | 4 |
| Lipids | <1 |
| Phosphorus | <1 |

(12) Structure:
Peptidoglycane structure in which the glycane structure consists of 10-80 disaccharides of N-acetylglucosaminyl-N-acetylmuramyl.

**2.** A process for the preparation of jomol according to Claim 1 by the cultivation of a strain of Nocardiaopaca-cells and recovery of the cells from the culture, characterised in that
(a) Norcardia-opaca-cells (DSM 43 202 or ATCC 21953) are cultivated on a buffered nutrient medium,
(b) the cells are recovered,
(c) the cells are suspended in a medium containing glucose and buffer and the suspension is left to stand for a period of from 15 minutes to several hours,

(d) the suspension is treated in a buffer with a murolytic enzyme, preferably lysozyme, and optionally with desoxyribonuclease,
(e) the cells are destroyed,
(f) the material obtained is separated into a ground sediment and a supernatant layer,
(g) the supernatant layer is subjected to separation in Sephadex columns, using, as eluting agent, a buffer which may contain from 0.1 to 0.05% of EDTA-$Na_2$,
(h) the active fraction is recovered and
(i) the active fraction is reacted with acetaldehyde,

for which the buffers used may be potassium phosphate buffers, tris-HCl buffers, ammonium acetate buffers and others.

**3.** A process according to Claim 2, characterised in that cultivation of the bacteria is preferably started on glucose agar (2%) dishes for 5 to 8 days and nutrient broth is used as nutrient medium in stage (a), and cultivation is carried out for a period of from 10 to 20 hours at a temperature of from 30 to 37° C.

**4.** A process according to Claim 2 or 3, characterised in that washing is carried out one or more times with tris-HCl buffer, pH 7.4, optionally containing from 0.1 to 0.05% EDTA-$Na_2$ after stage (b) and before incubation with glucose.

**5.** A process according to one of the Claims 2, 3 or 4, characterised in that the active fractions recovered are those which have an absorption at 214 nm in the UV spectrum.

**6.** A process according to one of the Claims 2, 3, 4 or 5, characterised in that the reaction of the active fraction with acetaldehyde is carried out in a molar ratio of 1 mol of active fraction (average molecular weight assumed to be 4000) with 1.8 to 2.2 mol of acetaldehyde.

**7.** Jomol, characterised in that it has been obtained according to one of the Claims 2 to 6.

**8.** Pharmaceutically acceptable jomol derivative, characterised in that it contains jomol according to Claim 1 and, coupled thereto, a crown ether, diethylene triaminopentacetic acid, uridine, L-thyronine, L-tyrosine, fluorescein isothiocyanate or tin(II) chloride.

**9.** Tagged jomol or tagged jomol derivative, characterised in that it contains jomol according to Claim 1 or one of its pharmaceutically acceptable derivatives according to Claim 8 tagged with $^{99m}$technetium, $^{111}$indium, $^{123}$iodine, $^{125}$iodine, $^{130}$iodine, $^{131}$iodine, $^{132}$iodine or $^{224}$radium, a dye or a cytostatic agent.

**10.** Jomol according to Claim 1, pharmaceutically acceptable jomol derivative according to Claim 8, tagged jomol or tagged jomol derivative according to Claim 9, characterised in that it is present
(i) in and/or on liposomes
or
(ii) lipidised.

**11.** A process for the preparation of a pharmaceutically acceptable jomol derivative according to Claim 8, characterised in that a jomol solution in a polar solvent is reacted with an anhydrous solution or suspension, a reactive precursor of a crown ether, diethylenetriaminopentacetic acid anhydride, a reactive precursor of uridine, L-thyronine, L-tyrosine or fluoroscein isothiocyanate or an aqueous hydrochloric acid solution of tin(II) chloride at a temperature in the range of from 15 to 40° C for a period of from 5 minutes to 60 minutes and the product is purified and isolated by chromatography in known manner.

**12.** A process for the preparation of tagged jomol or a tagged jomol derivative according to Claim 9, characterised in that a jomol solution is reacted in a polar solvent with a solution of a radioactive alkali metal iodide in the presence of Chloramine T and the solution obtained is optionally purified or in that a jomol solution in a polar solvent is reacted with a solution or suspension of a reactive precursor of a dye or cytostatic agent in an anhydrous solvent at a temperature of from 15 to 40° C for a period of from 5 minutes to 60 minutes or in that an aqueous solution of a jomol derivative according to Claim 8 is tagged in known manner with $^{99m}$technetium,$^{111}$indium, $^{123}$iodine, $^{125}$iodine, $^{130}$iodine, $^{131}$iodine, $^{132}$iodine or $^{224}$radium.

**13.** Diagnostic agent, characterised in that it contains jomol according to Claim 1 or 7 or jomol or a jomol derivative according to Claim 8 tagged with a radioactive isotope and/or a dye, and optionally conventional carriers and/or diluents.

**14.** Diagnostic agent according to Claim 13, characterised in that the jomol or jomol derivative is tagged with $^{99m}$technetium, $^{111}$indium, $^{123}$iodine, $^{125}$iodine, $^{130}$iodine, $^{131}$iodine, $^{132}$iodine or $^{224}$radium as radioactive isotope.

**15.** Pharmaceutical agent, characterised in that it contains jomol according to Claim 1 or tagged jomol or a tagged jomol derivative according to Claim 9 tagged with a dye, a cytostatic agent or a radioactive isotope, and optionally conventional carriers and/or diluents.

**16.** Pharmaceutical agent according to Claim 15, characterised in that the jomol or jomol derivative is tagged with $^{99m}$technetium, $^{111}$indium, $^{123}$iodine, $^{125}$iodine, $^{130}$iodine, $^{131}$iodine, $^{132}$iodine or $^{224}$radium as radioactive isotope.

**17.** Product containing

(1) a diagnostic agent according to Claim 13 or 14 or a pharmaceutical agent according to Claim 15 or 16 and

(2) an auxiliary agent containing an aldehyde corresponding to Formula I

RCHO (I)

wherein R denotes a hydrogen atom or a straight chain or branched hydrocarbon group having 1 to 4 carbon atoms, the free aldehyde being optionally released metabolically from substances either directly or indirectly and

optionally an alcohol corresponding to Formula II

$R^1CH_2OH$ (II)

wherein $R^1$ has the meaning indicated above for R

and optionally conventional pharmaceutically acceptable carriers and/or diluents,

for use, either simultaneously or separately or at time intervals, for the diagnosis and therapy of malignant tumours and for the therapy of immune deficiencies.

**18.** A product according to Claim 17, characterised in that it comprises two containers, component (1) being present in the first container and component (2) in the second container.

**19.** A product according to Claim 17, characterised in that R and $R^1$ in Formulae I and II denote a methyl group.

**20.** A product according to Claim 17, 18 or 19, characterised in that the auxiliary agent contains the aldehyde and the alcohol in a ratio of 15 to 40 g of aldehyde to 1000 g of alcohol.

**21.** A product according to one of the Claims 17 to 20, characterised in that compound (1) is present in a suitable form for oral or parenteral administration and the auxiliary agent (2) is present in a form suitable for oral or parenteral administration.

**22.** A product according to one of the Claims 17 to 20, characterised in that compound (1) is present in a form suitable for administration by inhalation and the auxiliary agent (2) is present in a form suitable for oral administration.

**23.** A product according to one of the Claims 17 to 22, characterised in that it comprises a third container with a component containing a pharmaceutically acceptable carrier.

**24.** A product according to Claim 23, characterised in that component (2) in addition contains a peroxide such as hydrogen peroxide.

## Revendications

1. Jomol, caractérisé par les propriétés suivantes :
   (1) maximum d'absorption dans le spectre UV à 282,5 nm ;
   (2) poids moléculaires apparents à 4 500, 3 000 et 900 ;
   (3) hygroscopicité élevée ;
   (4) selon la teneur en eau résiduelle, couleur ivoire, amorphe, cristallin, floconneux ;
   (5) pH de 5,5 (1 mg du composé, dissous dans 1 ml d'eau) ;
   (6) réaction à la nihydrine rose, légèrement positive ;
   (7) colorable par le réactif orcine ;
   (8) pourcentage de peptides : 50 % environ, par rapport à la substance amorphe
   (test des protéines MicroBioRad selon L. Thomas, J. Clin. Chem. Clin. Biochem., vol. 19, 1981, pages 203-208, Société BioRad, Munich ; bleu brillant de Coomassie G 250®) ;
   (9) valeurs $R_f$ suivantes (solvants indiqués en volume : volume)

| | |
|---|---|
| $R_f$ = 0,20 | (Butanol : acide acétique cristallisable : eau 4:1:1) |
| $R_f$ = 0,45 | (Benzène : acide acétique cristallisable : eau 2:1:2) |
| $R_f$ = 0,685 | (Méthanol : acide acétique cristallisable : eau 4:1:1) |
| pas de migration | (Chloroforme : méthanol 96:4) |
| $R_f$ = 0,54 | (Chloroforme : méthanol 1:1) ; |

   (10) solubilité : peu soluble dans les solvants non polaires, très soluble dans les solvants polaires ;
   (11) composants identifiables :

| Substances | Rapport (approximation) |
|---|---|
| Sucres neutres (glucose, galactose, ribose) | (≦) 1 |
| Sucres aminés (glucosamine, acide muramique) (après hydrolyse) | 3,5 |
| Acides aminés (Ala, Glu, iso-Gln, Gly, Lys, DAP) | 4 |
| Lipides | <1 |
| Phosphore | <1 |

   (12) structure :
   Structure de peptidoglycanne dont le squelette glycanne est composé de 10 à 80 disaccharides de N-acétylglucosaminyl-N-acétylmuramyle.

2. Procédé de production de Jomol selon la revendication 1 par culture d'une souche de cellules Nocardia-opaca et recueil des cellules de la culture, caractérisé en ce que :
   (a) on cultive des cellules Nocardia-opaca (DSM 43 202, soit ATCC 21953) sur un milieu nutritif tamponné,
   (b) on recueille les cellules,
   (c) on met les cellules en suspension dans un milieu contenant du glucose et du tampon, on laisse reposer la suspension pendant 15 min à quelques heures,
   (d) on traite la suspension dans un tampon avec une enzyme murolytique, de préférence un lysozyme, et le cas échéant avec une désoxyribonucléase,
   (e) on détruit les cellules,
   (f) on sépare le matériel obtenu en dépôt et surnageant,
   (g) on soumet le surnageant à la séparation sur des colonnes Sephadex en utilisant comme éluant un tampon contenant le cas échéant 0,1 à 0,05 % d'EDTA-$Na_2$,
   (h) on recueille la fraction active, et
   (i) on met à réagir la fraction active avec de l'acétaldéhyde,
   le tampon utilisable étant un tampon phosphate de potassium, un tampon Tris-HCl, un tampon acétate d'ammonium, ainsi que d'autres.

3. Procédé selon la revendication 2, caractérisé en ce qu'on cultive de préférence les bactéries pendant 5 à 8 jours sur des plaques d'agar (2 %) au sucre de raisin et qu'on utilise au cours de l'étape (a) un bouillon de culture comme milieu nutritif et qu'on procède à la culture pendant une durée de 10 à 20 h

à une température comprise entre 30 et 37 ° C.

4. Procédé selon la revendication 2 ou 3, caractérisé en ce qu'on procède à un ou plusieurs lavages avec un tampon Tris-HCl, pH 7,4, contenant le cas échéant 0,1 à 0,05 % d'EDTA-$Na_2$ après l'étape (b) et avant l'incubation avec le glucose.

5. Procédé selon une des revendications 2, 3 ou 4, caractérisé en ce qu'on recueille comme fractions actives celles présentant une absorption à 214 nm dans le spectre UV.

6. Procédé selon une des revendications 2, 3, 4 ou 5, caractérisé en ce qu'on procède à la réaction entre la fraction active et l'acétaldéhyde pour un rapport molaire de 1 mol de fraction active (poids moléculaire moyen supposé : 4 000) pour 1,8 à 2,2 mol d'acétaldéhyde.

7. Jomol, caractérisé en ce qu'il a été obtenu selon une des revendications 2 à 6.

8. Dérivé pharmaceutiquement acceptable du Jomol, caractérisé en ce qu'il contient du Jomol selon la revendication 1, auquel est couplé un éther couronne, de l'acide diéthylènetriaminepentaacétique, de l'uridine, de la L-thyronine, de la L-tyrosine, de l'isothiocyanate de fluorescéine ou du chlorure d'étain (II).

9. Jomol marqué ou dérivé marqué de Jomol, caractérisé en ce qu'il contient du Jomol selon la revendication 1 ou un de ses dérivés pharmaceutiquement acceptables selon la revendication 8, marqué par le technétium$^{99m}$ , l'indium$^{111}$ , l'iode$^{123}$ , l'iode$^{125}$, l'iode$^{130}$, l'iode$^{131}$, l'iode$^{132}$, ou le radium$^{224}$, un colorant ou un cytostatique.

10. Jomol selon la revendication 1, dérivé pharmaceutiquement acceptable du Jomol selon la revendication 8, Jomol marqué ou dérivé marqué du Jomol selon la revendication 9, caractérisé en ce qu'il est présent
   (i) dans et/ou sur des liposomes,
   ou
   (ii) dispersé dans un lipide.

11. Procédé de préparation d'un dérivé pharmaceutiquement acceptable du Jomol selon la revendication 8, caractérisé en ce qu'on met à réagir une solution de Jomol dans un solvant polaire avec une solution ou suspension anhydre d'un précurseur réactif d'un éther couronne, d'un anhydride diéthylènetriaminepentaacétique, d'un précurseur réactif d'uridine, de L-thyronine, de L-tyrosine ou d'isothiocyanate de fluorescéine ou d'une solution aqueuse chlorhydrique de chlorure d'étain (II) à une température comprise dans une plage de 15 à 40 ° pendant une durée comprise entre 5 min et 60 min et qu'on purifie et isole le produit par chromatographie d'une façon connue en soi.

12. Procédé de préparation de Jomol marqué ou d'un dérivé marqué du Jomol selon la revendication 9, caractérisé en ce qu on met à réagir une solution de Jomol dans un solvant polaire avec une solution d'un iodure radioactif d'une base en présence de chloramine T et qu'on purifie le cas échéant la solution obtenue ou qu'on met à réagir une solution de Jomol dans un solvant polaire avec une solution ou suspension d'un précurseur réactif d'un colorant ou d'un cytostatique dans un solvant anhydre à une température comprise dans une plage de 15 à 40 ° C pendant une durée de 5 min à 60 min ou qu'on marque une solution aqueuse d'un dérivé de Jomol selon la revendication 8 par le technétium$^{99m}$,l'indium$^{111}$, l'iode$^{123}$, l'iode$^{125}$, l'iode$^{130}$, l'iode$^{131}$ , l'iode$^{132}$ ou le radium$^{224}$ d'une façon connue en soi.

13. Outil de diagnostic, caractérisé en ce qu'il contient du Jomol selon la revendication 1 ou 7 ou du Jomol ou un dérivé du Jomol selon la revendication 8, marqué par un radioisotope et/ou un colorant, ainsi que, le cas échéant, des véhicules et/ou diluants usuels.

14. Outil de diagnostic selon la revendication 13, caractérisé en ce que le Jomol ou le dérivé de Jomol est marqué par le technétium$^{99m}$, l'indium$^{111}$, l'iode$^{123}$, l'iode$^{125}$, l'iode$^{130}$, l'iode$^{131}$, l'iode$^{132}$ ou le radium$^{224}$ en guise de radioisotope.

**15.** Médicament, caractérisé en ce qu'il contient du Jomol selon la revendication 1 ou du Jomol marqué ou un dérivé marqué du Jomol selon la revendication 9, marqué par un colorant, un cytostatique ou un radioisotope, ainsi que, le cas échéant, les véhicules et/ou diluants usuels.

**16.** Médicament selon la revendication 15, caractérisé en ce que le Jomol ou le dérivé du Jomol est marqué le technetium$^{99m}$,l'indium$^{111}$, l'iode$^{123}$, l'iode$^{125}$, l'iode$^{130}$, l'iode$^{131}$, l'iode$^{132}$ ou le radium$^{224}$ en guise de radioisotope.

**17.** Produit contenant :

(1) un outil de diagnostic selon la revendication 13 ou 14 ainsi qu'un médicament selon la revendication 15 ou 16 et

(2) un adjuvant qui contient un aldéhyde de formule I :

RCHO (I)

dans laquelle R désigne un atome d'hydrogène et un groupement hydrocarbure linéaire ou ramifié possédant 1 à 4 atomes de carbone, l'aldéhyde libre pouvant être libéré directement ou indirectement de substances par voie métabolique, et

le cas échéant, un alcool de formule II :

$R^1CH_2OH$ (II)

dans laquelle $R^1$ possède la signification indiquée ci-dessus pour R, ainsi que, le cas échéant, des véhicules et/ou diluants usuels, pharmaceutiquement acceptables, à utiliser simultanément, séparément ou à de certains intervalles dans le diagnostic et le traitement de tumeurs malignes et pour le traitement des déficits immunitaires.

**18.** Produit selon la revendication 17, caractérisé en ce qu'il comprend deux récipients, le composant (1) étant présent dans le premier récipient et le composant (2) étant présent dans le second récipient.

**19.** Produit selon la revendication 17, caractérisé en ce que R et $R^1$ dans les formules I et II désignent un groupement méthyle.

**20.** Produit selon la revendication 17, 18 ou 19, caractérisé en ce que l'adjuvant contient l'aldéhyde et l'alcool dans un rapport de 15 à 40 g d'aldéhyde pour 1 000 g d'alcool.

**21.** Produit selon une des revendications 17 à 20, caractérisé en ce que le composé (1) est présent sous une forme convenant à l'administration orale ou parentérale et que l'adjuvant (2) est présent sous une forme convenant à l'administration orale ou parentérale.

**22.** Produit selon une des revendications 17 à 20, caractérisé en ce que le composé (1) est présent sous une forme convenant à l'administration par inhalation et que l'adjuvant (2) est présent sous une forme convenant à l'administration orale.

**23.** Produit selon une des revendications 17 à 22, caractérisé en ce qu'il comprend un troisième récipient renfermant un composant qui contient un véhicule pharmaceutiquement acceptable.

**24.** Produit selon la revendication 23, caractérisé en ce que le composant (2) contient en outre un peroxyde, par exemple un peroxyde d'hydrogène.

FIG.1

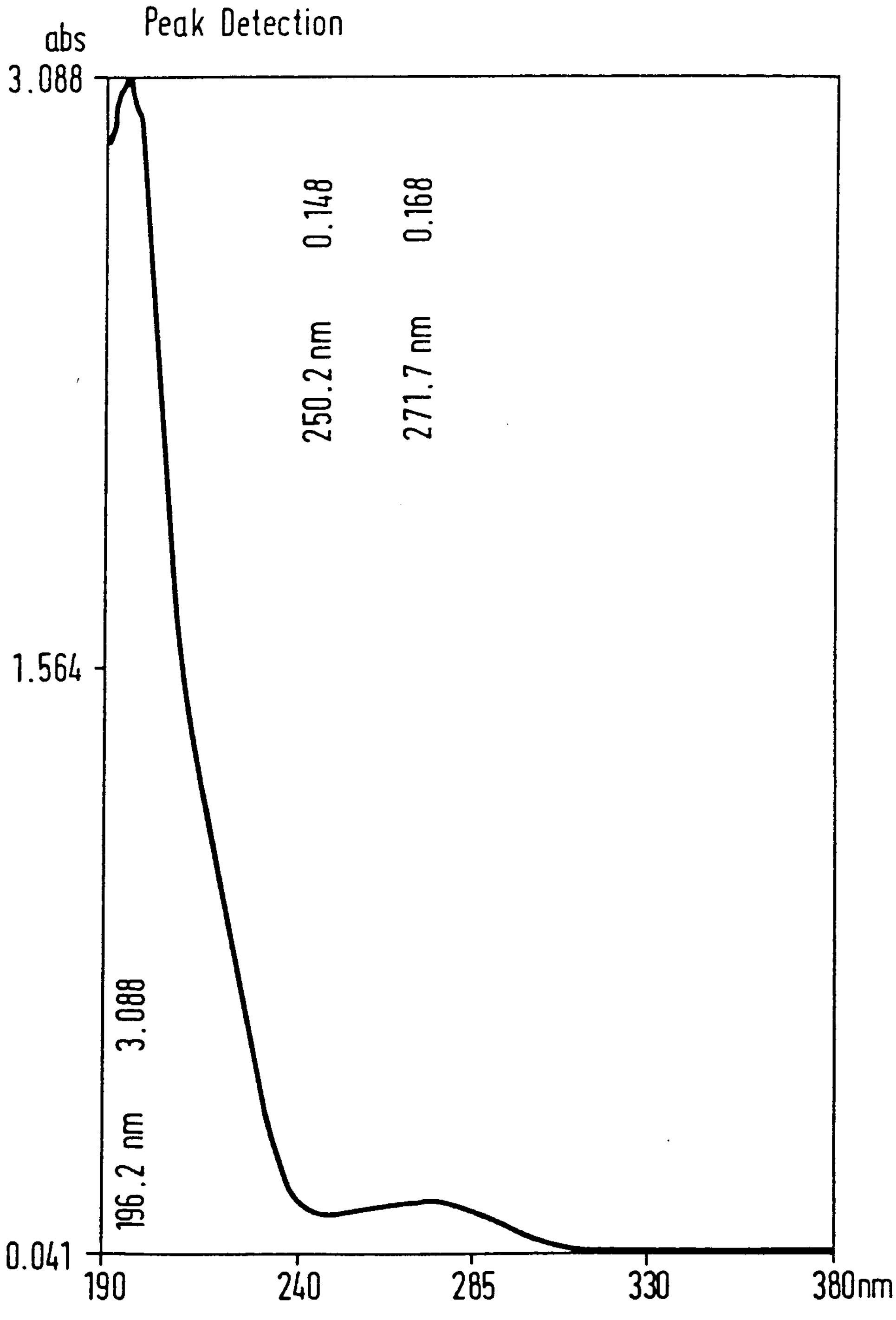


UV-Spektrum des Rohpräparates G2 aus Nocardia opaca vor Fraktionierung über Sephadex G 75

FIG . 2

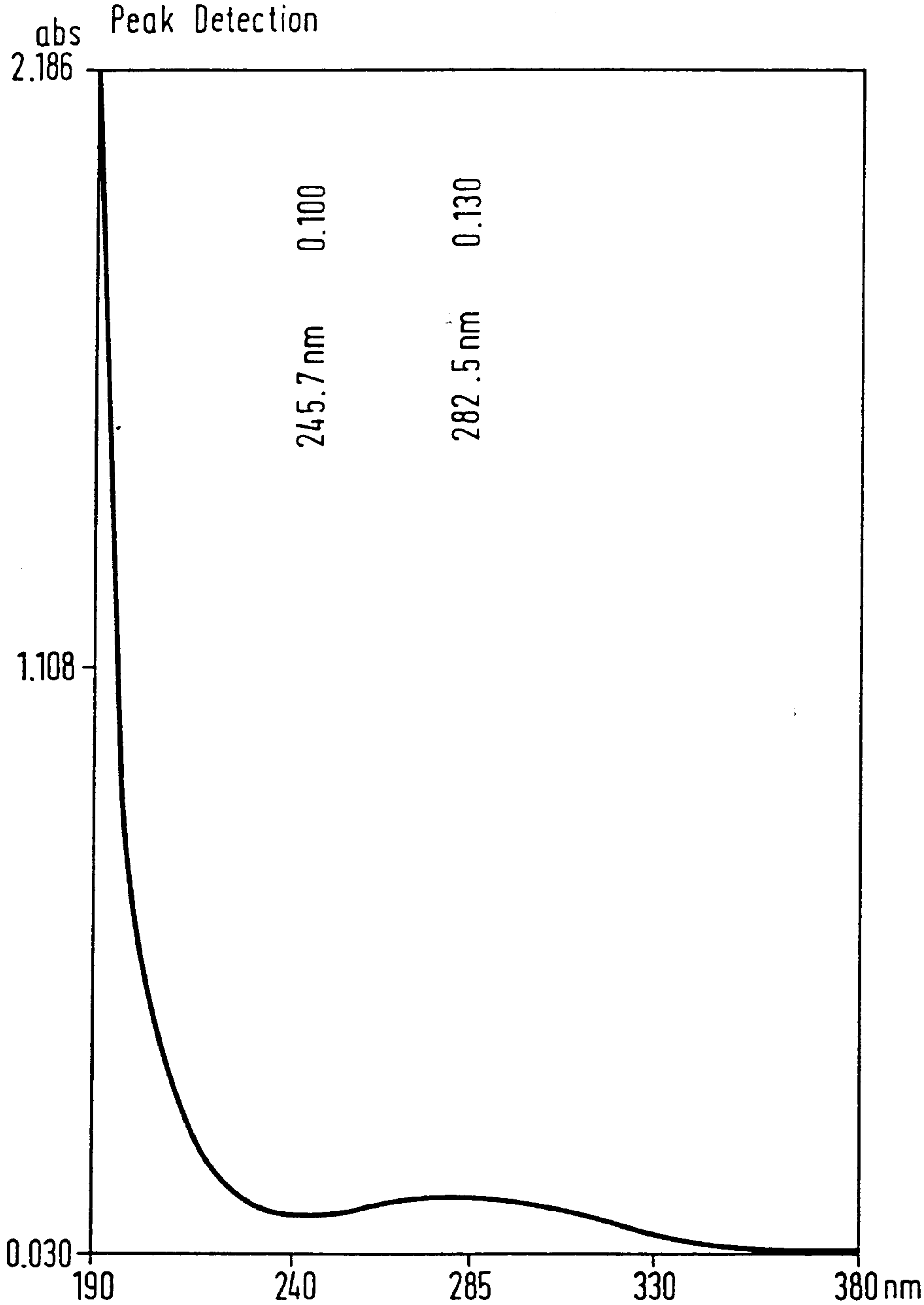


UV-Spektrum von Jomol (Fraktion 2b), Präparation G3, Frkt. 5

FIG.3a

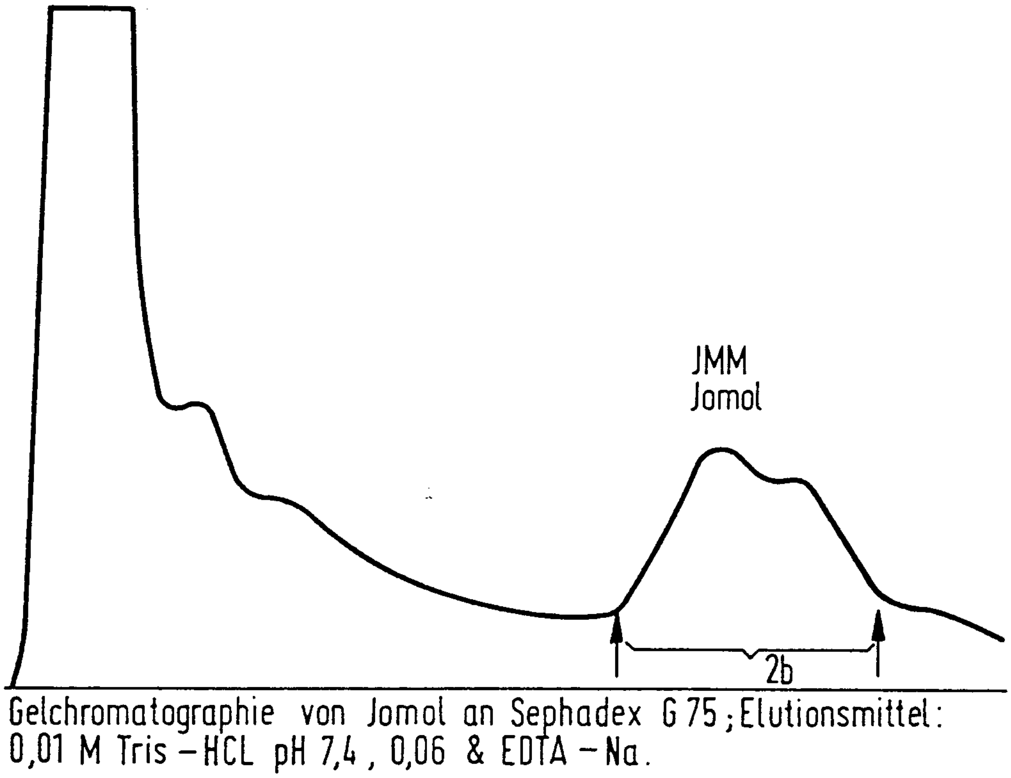


Gelchromatographie von Jomol an Sephadex G 75; Elutionsmittel: 0,01 M Tris-HCL pH 7,4, 0,06 & EDTA-Na.

FIG.3b

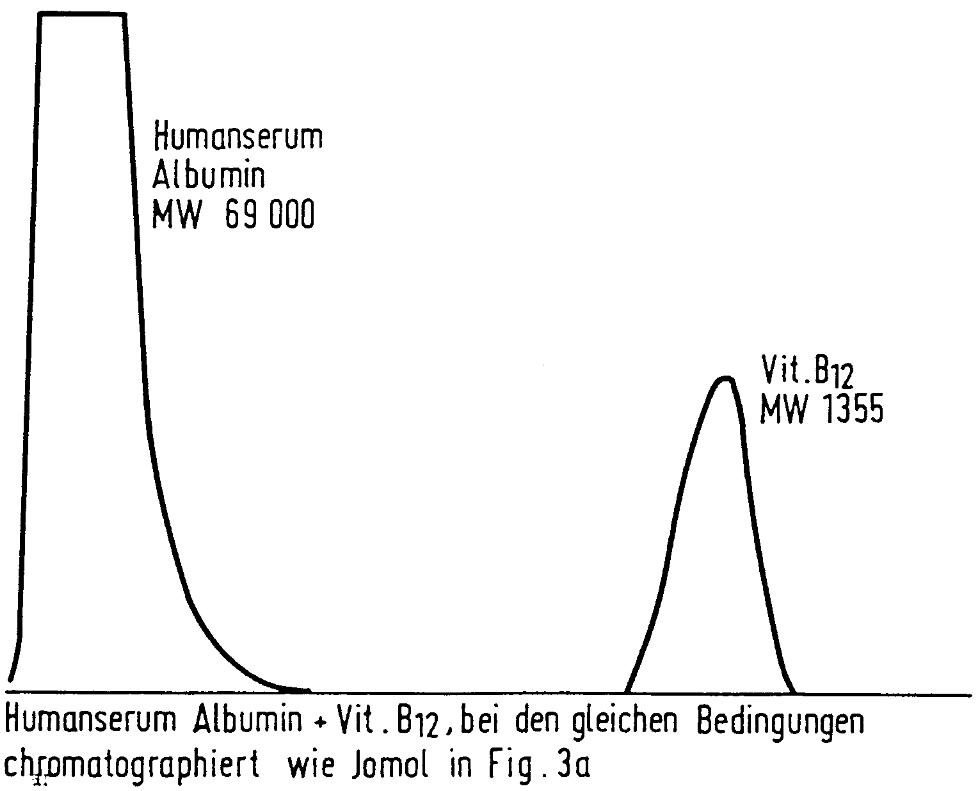


Humanserum Albumin + Vit. $B_{12}$, bei den gleichen Bedingungen chromatographiert wie Jomol in Fig. 3a

FIG. 4

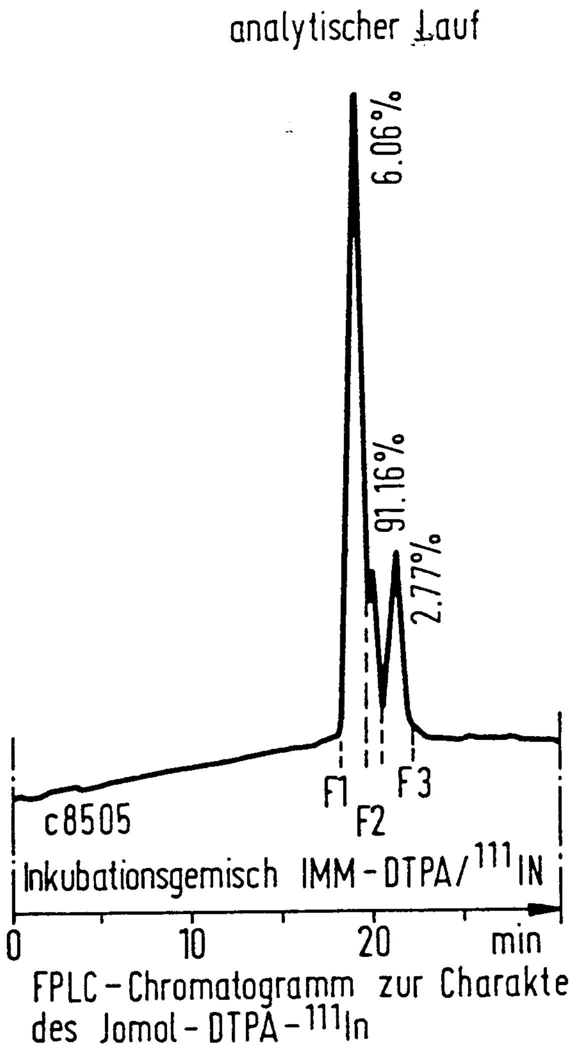


FPLC-Chromatogramm zur Charakterisierung des Jomol-DTPA-$^{111}$In